# EUROPÄISCHE PATENTSCHRIFT

(11) **EP 0 697 405 B1**
(45) Veröffentlichungstag und Bekanntmachung des Hinweises auf die Patenterteilung: **14.08.2002**
(21) Anmeldenummer: 95112585.5
(22) Anmeldetag: 10.08.1995
(51) Int. Cl.: C07D 215/227, C07D 215/36, C07D 215/38, A61K 31/47

(54) **Substituierte-Chinolinderivate, Verfahren zu ihrer Herstellung und ihre Verwendung**
Substituted quinoline derivatives, process for their preparation and their use
Dérivés de quinoléines substituées, procédé pour leur préparation et leur utilisation

(30) Priorität: 16.08.1994 DE 4428932
(43) Veröffentlichungstag der Anmeldung: 21.02.1996
(73) Patentinhaber: Aventis Pharma Deutschland GmbH, 65929 Frankfurt am Main (DE)
(72) Erfinder: Kirsch, Reinhard, Dr., D-38100 Braunsschweig (DE); Kleim, Jörg-Peter, Dr., D-65779 Kelkheim (DE); Riess, Günther, Dr., D-65795 Hattersheim (DE); Rösner, Manfred, Dr., D-65817 Eppstein (DE); Winkler, Irvin, Dr., D-65835 Liederbach (DE)

(56) Entgegenhaltungen:
- EP-A- 0 579 968
- WO-A-92/16508
- DE-A- 4 142 322
- J. ORG. CHEM. , Bd. 56, Nr. 18, 1991 Seiten 5311-5318, F. LEWIS ET AL. 'Spectroscopy and Photochemistry of 2-Quinolones and Their Lewis Acid Complexes'
- CHEM. BER., Bd. 122, Nr. 6, 1989 Seiten 1161-1173, G. HIMBERT ET AL. 'Einflu von Substituenten in p-, m- und o-Position am Aromaten auf die Intramolekulare Diels-Alder-Reaktion von Allencarbonsäure-aniliden und-phenylestern'

## Beschreibung

Gegenstand der vorliegenden Erfindung sind Alkylen-substituierte Chinolinderivate, Verfahren zu ihrer Herstellung und ihre Verwendung

Virale Infektionen bei Mensch und Tier, insbesondere beim Menschen, sind weit verbreitet. Trotz intensiver Bemühungen ist es bislang jedoch noch nicht gelungen, Chemotherapeutika aufzufinden, die ursächlich oder symtomatisch mit dem viral oder retroviral bedingten Krankheitsgeschehen mit einem erkennbar substantiellem Erfolg interferieren. Eine Behandlung viraler und insbesondere retroviraler Erkrankungen mittels Chemotherapeutika ist daher nur sehr unvollkommen. Aufgrund der weltweit stark ansteigenden Zahl an Personen, welche mit dem HIV-Virus infiziert sind, stellt insbesondere diese Art der retroviralen Virusinfektion ein weltweit wachsendes Problem dar.

Das als humanes Immundefizienzvirus (HIV) bezeichnetes Retrovirus wird i.a. als Verursacher der komplexen Krankheit, welche man als AIDS (Acquired Imune Deficiency Syndrome) bezeichnet, angenommen. AIDS verursacht eine progressive Zerstörung des Immunsystems des Erkrankten, verbunden mit einer Zerstörung des periphären und des zentralen Nervensystems. Ein wichtiger Schritt im Replikationszyklus von Retroviren ist die reverse Transcription des RNA-Genoms des Virus durch das viruseigene Enzym Reverse Transcriptase, welche DNA-Kopien der HIV-Sequenz liefert. Es ist bekannt, daß einige Verbindungen, wie z.B. Azidothymidin (AZT), als Inhibitoren der Reversen Transcriptase fungieren können. Sie werden daher zur Behandlung von AIDS angewendet. AZT und ähnliche Verbindungen vom Nukleosidtyp wie DDC oder DDI sind jedoch durch eine sehr enge therapeutische Breite bzw. durch bereits im therapeutischen Bereich auftretende, sehr schwere Toxizitäten charakterisiert (s. z.B. Hirsch, M.S. (1988) J. Infect. Dis. 157, 427 - 431). Des weiteren ist das Problem der Resistenzbildung gegen Chemotherapeutika noch ungelöst.

Iminochinolinderivate mit antiviraler Wirksamkeit gegen das HIV-Virus sind in der Patentanmeldung EP 93 109 965.9 beschrieben. Chinoxalinon-Derivate mit verwandten Strukturen mit antiviraler Wirksamkeit sind in der Patentanmeldung EP 93 109 965.9 beschrieben. Des weiteren sind die Verbindungen A und B bekannt (s. Kaneko, C. et al., Chem. Pharm. Bull., 17 (1969), 1290-1294 und Searles und Kelly, J.Am.Chem. Soc., 78 (1956),2242 - 2243); eine antivirale Wirkung dieser beiden Derivate ist jedoch nicht beschrieben.

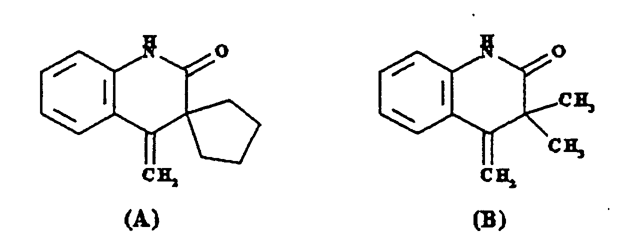

Nicht beschrieben sind weiterhin antiviral-wirksame Derivate, die sich von den genannten Iminochinolinverbindungen dadurch unterscheiden, daß sich in 4-Position des heterozyklischen Chinolin-Ringsystems über eine C-C-Doppelbindung gebundene Substituenten befinden.

Es wurde nun überraschenderweise gefunden, daß bestimmte 4-Alkylensubstituierte Chinolinderivate eine hohe antivirale Wirksamkeit insbesondere gegen das humane Imundefizienzvirus (HIV) aufweisen.

Erfindungsgegenstand sind demzufolge Verbindungen der Formel I, 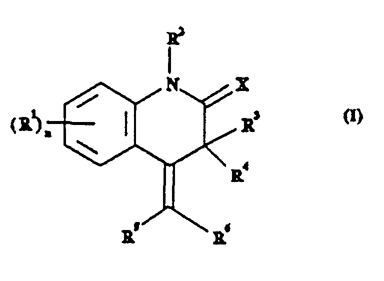 sowie deren tautomere Formen, der allgemeinen Formel Ia, 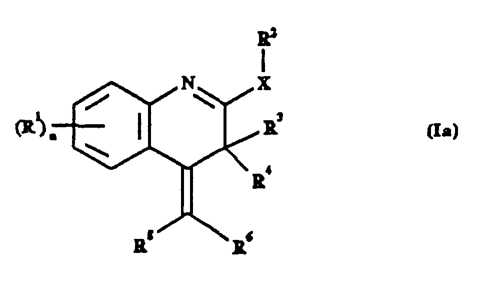 worin bedeuten:
1)
n
null,
eins,
oder zwei,
die einzelnen Substituenten R¹ unabhängig voneinander Fluor, Chlor, Trifluormethoxy, Hydroxy, C₁-C₄-Alkoxy, C₁-C₄-Alkylthio,
X Sauerstoff, Schwefel oder substituierten Stickstoff N-R², N-O-R²,

wobei R² Wasserstoff bedeutet;
R⁵ und R⁶ gleich oder verschieden, unabhängig voneinander Wasserstoff,
C₁-C₆-Alkyl, gegebenenfalls substituiert mit Fluor, Chlor, Amino, Mercapto, Hydroxy, C₁-C₄-Acyloxy, Benzoyloxy, Benzyloxy, Phenoxy, C₁-C₄-Alkoxy, C₁-C₄-Alkylamino. Di(C₁-C₄-alkyl)amino, C₁-C₄-Alkylthio;
C₂-C₆-Alkenyl, gegebenenfalls substituiert mit Fluor, Chlor, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio;
wobei mindestens ein Substituent von R⁵ und R⁶ Wasserstoff ist und
R⁵ und R⁶ können auch gemeinsam einen Carbozyklus der Ringgröße C₅-C₆ bedeuten, welcher über die Doppelbindung mit dem Chinolinsystem verknüpft ist,
R³ und R⁴ gleich oder verschieden, unabhängig voneinander C₁-C₂-Alkyl;
R³ und R⁴ können auch in Strukturen der Formeln gemäß I und la gemeinsam einen Carbozyklus der Ringgröße C₄-C₆ bedeuten, welcher spiroartig mit dem Chinolinsystem verknüpft ist.

Ein weiterer Gegenstand der vorliegenden Erfindung ist die Verwendung von Verbindungen der Formel I wie unter 1) beschrieben, deren optische Isomere, Diastereomere in reiner Form oder in Form ihrer Mischungen und deren Additionsalze und Prodrugs, zur Anwendung als Arzneimittel.

Weiterhin gehört zum Gegenstand der vorliegenden Erfindung die Verwendung der unter 1) genannten Verbindungen zur Herstellung von Arzneimitteln zur Behandlung von Viruserkrankungen.

Die in den vorangegangenen Definitionen genannten Alkylgruppen können geradkettig oder verzweigt sein. Sofern nicht anders definiert, enthalten sie vorzugsweise 1-8, besonders bevorzugt 1-6, insbesondere 1-4 C-Atome. Beispiele sind die Methyl-, Ethyl-, Propyl-, 1-Methylethyl-, Butyl-, 1-Methylpropyl-, 2-Methylpropyl-, 1,1-Dimethylethylgruppe und ähnliche.

Die in den vorangegangenen Definitionen genannten Alkenylgruppen können geradkettig oder verzweigt sein und enthalten 1 bis 3 Doppelbindungen. Sofern nicht anders definiert, enthalten diese Gruppen vorzugsweise 2-8, insbesondere 2-6 C-Atome. Beispiele sind die 2-Propenyl-, 1-Methylethenyl-, 2-Butenyl-, 3-Butenyl-, 2-Methyl-2-propenyl-, 3-Methyl-2-butenyl-, 2,3-Dimethyl-2-butenyl-, 3,3-Dichlor-2-propenyl- und Pentadienylgruppe und ähnliche.

Die in den vorangegangenen Definitionen genannten Acylgruppen können aliphatisch, cycloaliphatisch oder aromatisch sein. Sofern nicht anders definiert, enthalten sie vorzugsweise 1-8, besonders bevorzugt 2-7 C-Atome. Beispielhafte Acylgruppen sind die Formyl-, Acetyl-, Chloracetyl-, Trifluoracetyl-, Hydroxyacetyl-, Glycyl-, Propionyl-, Butyryl-, Isobutyryl-, Pivaloyl-, Cyclohexanoyl- oder Benzoylgruppe.

Die obengenannten Substituenten R¹ bis R⁷ sind vorzugsweise 3-fach, besonders bevorzugt 2-fach, insbesondere einfach mit den jeweils angegebenen Substituenten substituiert.
Für die jeweiligen zusammengesetzten Substituentendefinitionen (wie z. B. Arylalkoxycarbonyl) sind die zuvor als bevorzugt beschriebenen Bereiche für die einzelnen Substituenten ebenfalls bevorzugt.
In Abhängigkeit von den verschiedenen Substituenten können Verbindungen der Formeln I und la mehrere asymmetrische Kohlenstoffatome besitzen. Gegenstand der Erfindung sind deshalb sowohl die reinen Stereoisomeren als auch Mischungen derselben, wie z. B. das zugehörige Racemat.
Die reinen Stereoisomeren der Verbindungen der Formeln I und la lassen sich durch bekannte Methoden oder in Analogie zu bekannten Methoden direkt herstellen oder nachträglich trennen.
Zum Gegenstand der vorliegenden Erfindung gehört weiterhin ein Verfahren zur Herstellung von Verbindungen der Formeln I) und Ia) wie oben unter 1) erläutert, dadurch gekennzeichnet, daß
A) zur Herstellung von Verbindungen der Formeln I mit X gleich Sauerstoff und la mit X wie unter 1) definiert - mit der Ausnahme von N-R² gleich N-H - und den Resten R¹, R², R³, R⁴, R⁵ und R⁶ wie unter 1) definiert, man eine Verbindung der Formel II und IIa, 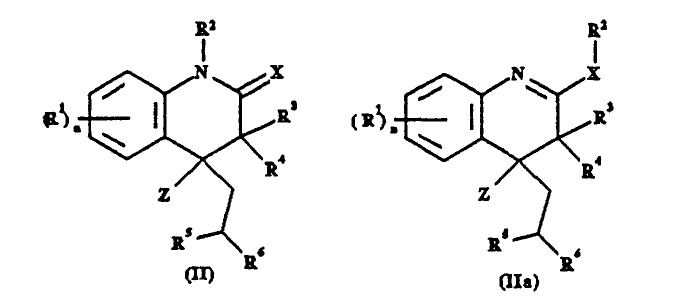 , wobei Z
   eine Abgangsgruppe oder eine Hydroxylgruppe darstellt, in einem inerten Lösungsmittel erhitzt, gegebenfalls unter Zusatz eines sauren oder basischen Katalysators.
B) zur Herstellung von Verbindungen der Formeln II mit X gleich Sauerstoff und Z gleich Hydroxyl und lla mit Z gleich Hydroxyl, X wie unter 1) definiert - mit der Ausnahme von N-R² gleich N-H -
   und den Resten R¹, R², R³, R⁴, R⁵ und R⁶ wie unter 1) definiert eine Verbindung der Formel III oder IIIa, 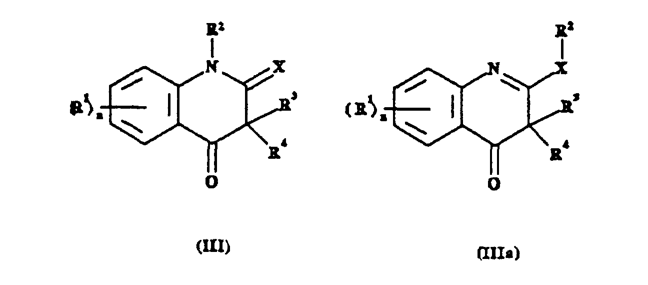 umsetzt mit einer Verbindung der Formel IV 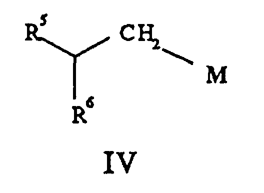 wobei M ein Metallatomäquivalent wie Li, -MgCI, -MgBr ist, oder
C) Zur Herstellung von Verbindungen der Formeln I mit X gleich Schwefel und R¹, R², R³, R⁴, R⁵ und R⁶ wie unter 1) definiert, durch Reaktion einer Verbindung der Formel I, , wobei X gleich Sauerstoff ist und für R¹, R², R³, R⁴, R⁵ und R⁶ die unter 1) genannten Definitionen gelten, mit einem Schwefelungsreagenz
   oder daß
D) Verbindungen der Formel I mit X gleich Sauerstoff, R² gleich Wasserstoff und R¹, R³, R⁴, R⁵ und R⁶ wie unter 1) definiert, hergestellt werden durch Reaktion mit einem Alkylierungsreagenz der Formel V

   R²-K, V

   wobei R² die unter 1) genannten Bedeutungen mit Ausnahme von R² gleich Wasserstoff aufweist und die Abgangsgruppe K beispielsweise ein Halogenatom wie Chlor oder Brom oder eine Sulfonsäureestergruppen wie Mesylat oder Triflat darstellt,
   oder daß
E) man eine Verbindung der Formel I mit R¹ - R⁶ wie unter 1) definiert und X ein Sauerstoff- oder Schwefelatom ist umsetzt mit einer Verbindung der Formel

   R²-NH₂ oder R²-O-NH₂

   zu Derivaten der Formel I mit R¹ - R⁶ wie unter 1) definiert und X gleich N-R² oder N-O-R²,
   oder daß
F) man eine Verbindung der Formel I mit R¹ - R⁶ wie unter 1) definiert und dabei einer dieser Reste eine Alkoxycarbonylgruppe besitzt, umsetzt mit einer Verbindung der Formel

   Met-OH,

   mit Met gleich einen Alkali- oder Erdalkalimetallatom ist, zu Derivaten der Formel I, die eine freie Carbonsäurefunktion aufweisen,
   oder daß
G) man eine Verbindung der Formel I mit R¹ = Methoxy und R² - R⁶ wie unter 1) definiert und X Sauerstoff umsetzt, mit Trimethylsilyliodid zu einer Verbindung der Formel I mit R¹ = Hydroxy und den Resten R² - R⁶ bzw. X wie oben definiert.

Die obengenannte Methode A verläuft vorzugsweise unter folgenden Bedingungen ab:
Als Abgangsgruppen Z seinen beispielhaft Halogen und Sulfonsäureestergruppen wie die Mesylat oder Triflat genannt.

Die Umsetzung wird zweckmäßigerweise in einem Lösungsmittel durchgeführt. Geeignet sind z. B. aromatische Kohlenwasserstoffe wie Toluol oder Xylol, Wasser, niedere Alkohole wie Methanol, Ethanol, Methylglycol oder 1-Butanol, Ether wie Tetrahydrofuran oder Glycoldimethylether, basische Lösungsmittel wie Pyridin oder N-Methylimidazol, Carbonsäuren wie Essigsäure oder Gemische dieser Lösungsmittel.
Die Anwesenheit eines geeigneten sauren oder basischen Katalysators z. B. p-Toluolsulfonsäure, Essigsäure, Mineralsäuren oder Salze wie Natriumacetat, Natriumcarbonat, Kaliumcarbonat oder Pyridiniumhydrochlorid ist günstig.
Die Reaktionstemperatur kann zwischen 0 und 200°C liegen, vorzugsweise bei der Siedetemperatur des Lösungsmittels.
Die obengenannte Methode B verläuft vorzugsweise unter folgenden Bedingungen:
Die Reaktionen werden zweckmäßigerweise in einem Lösungsmittel durchgeführt. Als Lösungsmittel eignen sich beispielsweise acyclische Dialkylether wie Diethylether oder Di-t-butylether oder cyclische Ether wie Tetrahydrofuran. Die Umsetzungen werden zweckmäßigerweise bei einer Temperatur von -78°C bis zur Siedetemperatur des jeweiligen Lösungsmittels vorzugsweise zwischen -30°C und +25°C durchgeführt. Im Allgemeinen wird bei einer Umsetzung nach der Methode B eine Verbindung der Formel III oder IIIa mit den Substituenten wie oben definiert mit mindestens 2 bis 10 vorzugsweise 2,2 bis 4 Moläquivalenten einer Verbindung der Formel IV vorzugsweise eine Grignardverbindung mit M gleich Magnesiumhalogenid zur Reaktion gebracht.

Für die Umsetzung wie zuvor unter C) beschrieben wird vorzugsweise als Schwefelungsreagenz 2,4-Bis(4-methoxyphenyl)-1,3-dithia-2,4-diphosphetan-2,4-disulfid (Lawessons Reagenz), Bis(tricyclohexylzinn)sulfid, Bis(tri-n-butylzinn)sulfid, Bis(triphenylzinn)sulfid, Bis(trimethylsilyl)sulfid oder Phosphorpentasulfid verwendet. Die Reaktion wird zweckmäßigerweise in einem organischen Lösungsmittel oder einem Lösungsmittelgemisch, bei Raumtemperatur oder höher, bevorzugt bei der Siedetemperatur des Reaktionsgemisches und möglichst unter wasserfreien Bedingungen durchgeführt. Geeignet sind z. B. Schwefelkohlenstoff, Toluol, Xylol, Pyridin oder 1,2-Dichlorethan. Bei Verwendung der erwähnten Zinn- oder Silylsulfide ist es angebracht, die Schwefelungsreaktion in Gegenwart einer Lewissäure wie Bortrichlorid durchzuführen.
In Gegenwart anderer Carbonylgruppen, sind diese gegebenenfalls vor der Schwefelungsreaktion nach bekannten Methoden durch eine geeignete Schutzgruppe, z. B. durch Acetalisierung, zu schützen.
Die oben genannte Methode D) wird im allgemeinen nach folgender Methode durchgeführt:

Die Umsetzung wird zweckmäßigerweise in einem Lösungsmittel durchgeführt. Geeignet sind z. B. aromatische Kohlenwasserstoffe wie Toluol oder Xylol, Wasser, niedere Alkohole wie Methanol, Ethanol, Methylglycol oder 1-Butanol, Ether wie Tetrahydrofuran oder Glycoldimethylether, basische Lösungsmittel wie Pyridin oder N-Methylimidazol oder Gemische dieser Lösungsmittel.

Die oben genannte Methode E) wird
im allgemeinen nach folgender Methode durchgeführt:

Die Umsetzung wird zweckmäßigerweise in einem Lösungsmittel durchgeführt. Geeignet sind z. B. aromatische Kohlenwasserstoffe wie Toluol oder Xylol, Wasser, niedere Alkohole wie Methanol, Ethanol, Methylglycol oder 1-Butanol, Ether wie Tetrahydrofuran oder Glycoldimethylether, basische Lösungsmittel wie Pyridin oder N-Methylimidazol oder Gemische dieser Lösungsmittel.

Die oben genannte Methode F) wird
im allgemeinen nach folgender Methode durchgeführt:

Die Umsetzung wird zweckmäßigerweise in einem Lösungsmittel durchgeführt. Geeignet sind z. B. Gemische aus Wasser und niederem Alkohol wie Methanol, Ethanol, Methylglycol oder 1-Butanol. Die Reaktionstemperatur kann zwischen 0 und 200°C liegen, vorzugsweise zwischen 20°C und der Siedetemperatur des verwendeten Lösungsmittelgemisches.

Die oben genannte Methode G) wird
im allgemeinen nach folgender Methode durchgeführt:

Die Umsetzung wird zweckmäßigerweise in einem Lösungsmittel durchgeführt. Geeignet sind z. B. halogenierte Kohlenwasserstoffe wie Chloroform, Dichlormethan, Tetrachlorkohlenstoff oder 1,2-Dichlorethan oder Gemische dieser Lösungsmittel. Die Reaktionstemperatur kann zwischen -20° und +200°C liegen, vorzugsweise zwischen +20°C und der Siedetemperatur des verwendeten Lösungsmittels.

Die Ausgangsmaterialien der allgemeinen Formeln III und IIIa sind literaturbekannt oder lassen sich nach literaturbeschriebenen Methoden herstellen (z.B. Patentanmeldung EP 93109965.9 sowie A. B. Daruwala et al., J. Med. Chem. 1974, 17, 819, G. M. Coppola, Synthesis 1980, 505 und hier zitierte Literatur) . Bespielsweise kann man bei der Synthese von Verbindungen des allgemeinen Typs III mit X gleich Sauerstoff von Isatosäureanhydriden der allgemeinen Formel VII ausgehen und bringt diese mit einer Verbindung der Formel VIII zur Reaktion. M stellt dabei ein Metallatom oder ein Metallatomäquivalent, vorzugsweise ein Erdalkali- oder Alkalimetall dar, vorzugsweise Lithium.

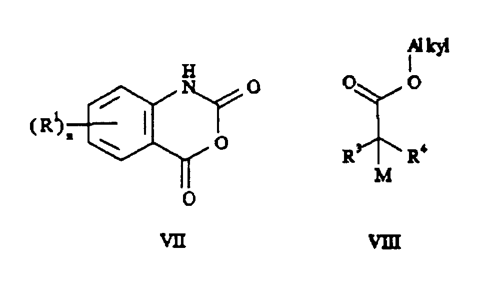

Die erfindungsgemäßen Arzneimittel können enteral (oral), parenteral (intravenös), rektal, subcutan, intramuskulär oder lokal (topisch) angewendet werden.
Sie können in Form von Lösungen, Pulvern (Tabletten, Kapseln einschließlich Microkapseln), Salben (Cremes oder Gele) oder Suppositorien verabreicht werden. Als Hilfsstoffe für derartige Formulierungen kommen die pharmazeutisch üblichen flüssigen oder festen Füllstoffe und Streckmittel, Lösemittel, Emulgatoren, Gleitstoffe, Geschmackskorrigentien, Farbstoffe und/oder Puffersubstanzen in Frage.
Als zweckmäßige Dosierung werden 0.1 - 10, vorzugsweise 0.2 - 8 mg/kg Körpergewicht ein- oder mehrmals täglich verabreicht. Die verwendeten Dosierungseinheiten richten sich zweckmäßigerweise nach der jeweiligen Pharmakokinetik der verwendeten Substanz bzw. der verwendeten galenischen Zubereitung.
Die verwendete Dosierungseinheit der erfindungsgemäßen Verbindungen beträgt z. B. 1 - 1500 mg, vorzugsweise 50 - 500 mg.
Die erfindungsgemäßen Verbindungen können auch in Kombination mit anderen antiviralen Mitteln, wie z. B. Nucleosidanaloga, Proteaseinhibitoren oder Adsorptionsinhibitoren und Immunstimulantien, Interferonen, Interleukinen und koloniestimulierenden Faktoren (z. B. GM-CSF, G-CSF, M-CSF) verabreicht werden.
Wirksamkeitstests
Prüfung von Präparaten gegen HIV in der Zellkultur
Methodenbeschreibung

### Medium:

### RPMI pH 6.8

Komplettes Medium enthält zusätzlich 20 % foetales Kälberserum und 40 IU/ml rekombinantes Interleukin 2.

### Zellen:

Aus frischem Spenderblut mittels Ficol^{R}-Gradienten-Zentrifugation isolierte Lymphozyten werden unter Zusatz von 2 g/ml Phytohämagglutinin (Wellcome) in komplettem Medium 36 h bei 37 C unter 5 % CO₂ kultiviert. Die Zellen werden nach Zusatz von 10 % DMSO bei einer Zelldichte von 5 x 10⁶ eingefroren und in flüssigem Stickstoff gelagert. Für den Versuch werden die Zellen aufgetaut, im RPMI-Medium gewaschen und im kompletten Medium 3 - 4 Tage kultiviert.

### Ansatz:

Die Prüfpräparate wurden in einer Konzentration von 16.7 mg/ml in DMSO gelöst und in komplettem Medium auf 1 mg/ml verdünnt.
In 24er Multiwell-Schalen wurden 0.4 ml Medium vorgelegt. Nach Zugabe von 0.1 ml des gelösten Präparates in die obere Reihe der Schale wurde durch Übertragung von jeweils 0.1 ml eine geometrische Verdünnungsreihe erzeugt. Präparatfreie Kontrollen enthielten stets 0.4 ml komplettes Medium mit 0.5% DMSO.
Lymphozytenkulturen mit einer Zellzahl von 5 x 10⁵ Zellen/ml wurden durch Zugabe 1/50 Volumen Überstand aus HIV-infizierten Lymphozytenkulturen infiziert. Der Titer dieser Kulturüberstände wurde durch Endpunktverdünnung mit 1 - 5 x 10⁶ infektiöse Einheiten/ml bestimmt. Nach 30 min Inkubation bei 37°C wurden die infizierten Lymphozyten abzentrifugiert und im gleichen Volumen Medium wieder aufgenommen. Von dieser Zellsuspension wurden jeweils 0.6 ml in alle Vertiefungen der Testplatte gegeben. Die Ansätze wurden 3 Tage bei 37°C inkubiert.

### Auswertung:

Die infizierten Zellkulturen wurden unter dem Mikroskop auf Anwesenheit von Riesenzellen untersucht, die eine aktive Virusvermehrung in der Kultur anzeigen. Die geringste Präparatekonzentration, bei der keine Riesenzellen auftraten, wurde als Hemmkonzentration gegen HIV bestimmt (MHK-Wert). Zur Kontrolle wurden die Überstände aus den Kulturplatten mit Hilfe eines HIV-Antigentests entsprechend den Angaben des Herstellers (Organon) auf Anwesenheit von HIV-Antigen bestimmt.

### Ergebnisse:

Die Ergebnisse dieses Tests zeigt Tabelle 1.

**Tabelle 1**

| Verbindungsnummer | MHK | IC-50 |
|---|---|---|
| 1 | 1 µg/ml | > = 40 ng/ml |
| 2 | 0.1 µg/ml | ca. = .015 µg/ml |
| 3 | < 0.08µg/ml | 0.03µg/ml |
| 4 | 0,0016 µg/ml | < 0.32 nq/ml |
| 5 | 0.2 µg/ml | 0.04 µg/ml |
| 7 | < 0.008 µg/ml | ca. 0.00032 µg/ml |
| 8 | 0.008 µg/ml | 0.0016 µg/ml |
| 10 | <0.04 µg/ml | 0.025 µg/mg |
| 13 | > 0.2µg/ml | 0.040 µg/ml |
| 14 | <0.008µg/ml | 0.005 µg/ml |
| 16 | <0.2 µg/ml | ca. 0.03 µg/ml |
| 17 | < 0.02 µg/ml | ca. 0.040 µg/ml |
| 18 | < 0.008 µg/ml | 2 ng/ml |
| 19 | < 0.04 mncg/ml | 0.008 µg/ml |
| 20 | < 0.008 µg/ml | 0,0015 µg/ml |
| 28 | 0,008 µg/ml | ca. 0,01 µg/ml |
| 30 | < 0,2 µg/ml | ca. 20 ng/ml |
| 32 | 0,2 µg/ml | ca. 80 ng/ml |
| 34 | < 0,04 µg/ml | ca. 0,01 µg/ml |
| 36 | <0,2 µg/ml | ca. 10 ng/ml |
| 38 | < 0,2 µg/ml | ca. 80 ng/ml |
| 41 | 0,2 µg/ml | ca. 0,2 µg/ml |
| 42 | <0,008 µg/ml | 0,002 µg/ml |
| 43 | 0,2 µg/ml | 0,05 µg/ml |
| 44 | 0,2 µg/ml | 0,06 µg/ml |
| 45 | 0,004 µg/ml | 0,002 µg/ml |
| 47 | <0,08 µg/ml | ca. 0,015µg/ml |
| 48 | < 0,08 µg/ml | ca. 0,01 µg/ml |
| 49 | <0,02 µg/ml | ca. 0,002 µg/ml |
| 50 | <0,1 µg/ml | ca. 0,01 µg/ml |
| 51 | 0,02 µg/ml | ca. 0,002 µg/ml |
| 52 | 0,02 µg/ml | ca. 0,004 µg/ml |
| 53 | 0,02 µg/ml | ca. 0,002 µg/ml |
| 54 | 0,1 µg/ml | 0,05 µg/ml |
| 55 | <0,08 µg/ml | ca. 0,006 µg/ml |
| 58 | < 0,1 µg/ml | ca. 0,02 µg/ml |
| 59 | < 0,02 µg/ml | ca 0,008 µg/ml |
| 60 | 0,0016 µg/ml | ca. 0,0003 µg/ml |
| 61 | 0,02 µg/ml | ca. 0,004 µg/ml |
| 66 | <0,1 µg/ml | ca. 0,01 µg/ml |
| 67 | <0,1 µg/ml | ca. 0,02 µg/ml |
| 69 | <0,02 µg/ml | ca. 0,0015 µg/ml |
| 70 | 0,02µg/ml | ca. 0,003 µg/ml |
| 71 | 0,0016µg/ml | ca. 0,0005 ng/ml |
| 72 | <0,008 µg/ml | ca. 0,001 ng/ml |
| 73 | <0,04 µg/ml | 0,02 µg/ml |
| 74 | 0,04 µg/ml | 0,02 µg/ml |
| 75 | 0,004 µg/ml | ca. 0,0018 µg/ml |
| 77 | 0,2 µg/ml | ca. 0,03 µg/ml |
| 138 | >0,2 µg/ml | 0.030 µg/ml |
| 140 | 0,2 µg/ml | 0,006 µg/ml |
| 142 | <0,2 µg/ml | 0,004 µg/ml |
| 143 | > 0,008 µg/ml | < 0,008 µg/ml |

Untersuchung der Substanzen auf Hemmung der HIV-"Reverse Transkriptase" Die Aktivität der Reversen Transkriptase (RT) wurde mit Hilfe eines "Scintillation Proximity Assay" (SPA) bestimmt.
Das Reagenzkit für den RT-SPA wurde von Amersham/Buchler (Braunschweig) bezogen. Das Enzym RT (aus HIV in E. coli cloniert) stammte von der Firma HT-Biotechnology LTD, Cambridge, UK.

### Ansatz:

Der Test wurde nach dem Methoden-Manual des Herstellers Amersham durchgeführt - mit folgenden Modifikationen:
- dem "Assay"-Puffer wurde Rinderserumalbumin zu der Endkonzentration 0.5 mg/ml zugesetzt.
- der Test wurde in Eppendorf-Reaktionsgefäßen mit 100 ml Ansatzvolumen durchgeführt.
- das RT-Konzentrat des Herstellers (5000 U/ml) wurde in Tris-HCI Puffer 20 mM; pH 7.2; 30 % Glycerin auf eine Aktivität von 15 U/ml verdünnt.
- die Inkubationszeit für die Ansätze betrug 60 min (37 C).
- nach Abstoppen der Reaktion und "Entwicklung" mit der Perlen-Suspension wurden 130 ml Ansatz in 4.5 ml Tris-HCI Puffer, 10 mM; pH 7.4; 0.15 M NaCI transferiert
und die Tritium-Aktivität in einem β-Counter gemessen.

### Substanzprüfung:

Für eine Vorprüfung der Inhibitoraktivität wurden die Substanzen in DMSO gelöst (Stammlösung c = 1 mg/ml) und in Verdünnung in DMSO 10⁻¹, 10⁻², 10⁻³ usw. getestet.
Zur Bestimmung von IC₅₀-Werten wurden die Inhibitor-Stammlösungen in Tris-HCI Puffer, 50 mM, pH 8 weiterverdünnt und in geeigneten Konzentrationen getestet.

Aus der graphischen Darstellung RT-Aktivität versus Log C_{Inh.} wurde die einer 50 %igen Enzymhemmung zugehörige Konzentration ermittelt.
Die Ergebnisse der Untersuchung zeigt Tabelle 2.

**Tabelle 2**

| Verbindungsnummer | Reverse Transkriptase Assay IC-50 |
|---|---|
| 2 | 17 ng/ml (63 nM) |
| 3 | 27ng/ml |
| 4 | 2 ng/ml (8 nM) |
| 5 | 115,5 ng/ml (479 nM) |
| 6 | 28,8 ng/ml (113 nM) |
| 7 | 5 ng/ml (18 nM) |
| 8 | 4 ng/ml (16 nM) |
| 10 | 31 ng/ml (116 nM) |
| 14 | 4 ng/ml (18 nM) |
| 18 | 15,2 nM (55 nM) |
| 19 | 10,3 ng/ml (37 nM) |
| 20 | 6,3 ng/ml (22 nM) |
| 24 | 66,3 ng/ml (270 nM) |
| 28 | 5,8 ng/ml (24 nM) |
| 42 | 2,6 ng/ml (9 nM) |
| 45 | 2,4 ng/ml (9 nM) |
| 48 | 14 ng/ml (54 nM) |
| 49 | 7 ng/ml (24 nM) |
| 51 | 6 ng/ml (23 nM) |
| 52 | 12 ng/ml (43 nM) |
| 53 | 13 ng/ml (44 nM) |
| 55 | 9 ng/ml (31 nM) |
| 59 | 14 ng/mg (46 nM) |
| 60 | 3 ng/ml (12 nM) |
| 61 | 17 ng/ml (62 nM) |
| 64 | 33 ng/ml (109 nM) |
| 66 | 19 ng/ml (70 nM) |
| 67 | 24 ng/ml (91 nM) |
| 68 | ca 10 ng/ml |
| 69 | 5 ng/ml (18 nM) |
| 70 | 6 nq/ml (21 nM) |
| 71 | 1 ng/ml ( 5 nM) |
| 73 | 8 ng/ml (30 nM) |
| 75 | < 1 ng/ml |
| 138 | 61 ng/ml |
| 140 | 5 ng/ml |
| 142 | 38 ng/ml |

Durch die nachfolgenden Beispiele sowie durch den Inhalt der Patentansprüche wird die vorliegende Erfindung näher erläutert.

### Herstellung der Ausgangsmaterialien

### Beispiel I

### 6-Chlor-3,3-dimethyl-1,3-dihydrochinolin-2,4-dion

Bei -70°C wird aus 7.3 g (0.072 mol) Diisopropylamin in 100 ml wasserfreiem Tetrahydrofuran und 45 ml einer 1.6 M Lösung von n-Butyllithium in Hexan eine Lösung von 0.072 mol Lithiumdiisopropylamid hergestellt. Nach kurzem Erwärmen auf -20°C werden bei -70 °C 3.48 g (0.03 mol) Isobuttersäureethylester zugegeben. Man läßt auf 0°C erwärmen und rührt 30 min bei dieser Temperatur. Die Lithiumverbindung wird anschließend zu einer auf -30°C gekühlten Suspension von 5.9 g (0.03 mol) 5-Chlorisatinsäureanhydrid in 50 ml wasserfreiem Tetrahydrofuran zugetropft. Man läßt das Reaktionsgemisch innerhalb einer Stunde auf 0°C erwärmen und gibt die gelbe Reaktionslösung auf 400 ml Eiswasser. Es wird dreimal mit Essigsäureethylester extrahiert, die vereinigten organischen Phasen je einmal mit gesättigter wäßriger Natriumhydrogencarbonat- und gesättigter wäßriger Natriumchloridlösung gewaschen, über Natriumsulfat getrocknet und eingeengt. Nach Verrühren mit Ether/ Pentan erhält man 5.1 g (76 %) der gewünschten Verbindung, Schmp. 211 - 212 C nach Kristallisation aus Isopropanol.
¹H-NMR (200 MHz, d₆-DMSO): = 1.30 (s, 6 H), 7.11 (d, J = 7.5 Hz, 1 H), 7.6 - 7.7 (m, 2 H), 10.87 ppm (s, 1H).
MS: (M + H)⁺ = 224

### Beispiel II

### 6-Chlor-3,3-diethyl-1,3-dihydrochinolin-2,4-dion

Eine Suspension von 22,1 g (0,088 mol) 3,3-Ethylmalonsäure-(4-chloranilid) in 220 g Polyphosphorsäure (ca. 84 % P₂O₅) wird unter kräftigem Rühren auf 80°C erwärmt und drei Stunden gerührt. Dabei löst sich die Substanz unter Gelbfärbung auf. Nach beendeter Reaktion wird das Reaktionsgemisch unter Rühren auf ca. 1000 ml Eiswasser gegossen, wobei sich das Produkt nach einiger Zeit als gelblich-weißer Feststoff abscheidet. Es wird abgesaugt, mit Wasser neutral gewaschen und unter Vakuum bei 50°C getrocknet.
Ausbeute: 12,6 g (61 %),
Schmelzpunkt 188°C (nach Umkristallisation aus Isopropanol/Heptan)
¹H-NMR (200 MHz, d₆-DMSO): δ = 1.27 (s, 6H), 1.29 (t, J = 7Hz, 3H), 4.03 (q, J = 7Hz,2H), 7.05 (d, J = 8Hz, 1H), 7.18 (m, 2H), 10.60 (br s, 1H)
MS: (M + H)⁺ = 234

### Beispiel III

### 3,3-Dimethyl-6-methoxy-1,3-dihydrochinolin-2,4-dion

Analog Beispiel II wurden unter Verwendung von 21.8 g (0.092 mol) 3,3-Dimethylmalonsäure-(4-methoxyanilid) 16.2 g (80 %) der gewünschten Verbindung erhalten. Schmp. 169 - 170°C (nach Umkristallisation aus Isopropanol)
¹H-NMR (200 MHz, d₆-DMSO): = 1.31 (s, 6 H), 3.78 (s, 3 H), 7.02 - 7.28 (m, 3 H), 10.62 ppm (s, 1 H).
MS: (M + H)⁺ = 220

### Herstellung der Endprodukte

### Beispiel 1

### 4-n-Butyl-6-chlor-3,4-dihydro-4-hydroxy-chinolin-2(1H)-on (V.43)

4,47 g (20 mmol) 6-Chlor-3,3-dimethyl-1,3-dihydrochinolin-2,4-dion (Beispiel I) werden in 100 ml absolutem Tetrahydrofuran gelöst und die Lösung auf -25°C gekühlt. Anschließend wird mit 30 ml (60mmol) einer 2M Lösung von n-Butylmagnesiumbromid in THF innerhalb von 30 Minuten versetzt. Nach Beendigung der Zugabe wird die Kühlung entfernt und das Reaktonsgemisch drei Stunden bei 25 °C gerührt.
Zur Aufarbeitung wird die Reaktionslösung auf 200 ml gesättigte wässrige Natriumchlorid-Lösung gegeben und das Gemisch mit 10-prozentiger wässr. Citronensäure-Lösung angesäuert (pH 3). Anschließend wird mit 100ml Essigsäureethylester dreimal ausgeschüttelt, die vereinigte organische Phase mittels Natriumsulfat getrocknet und unter reduziertem Druck am Rotationsverdampfer eingeengt. Das auf diese Weise erhaltene hellgelbe Öl (7,2 g) wird mittels Chromatographie an Kieselgel mobile Phase n-Heptan/Essigsäureethylester = 2/1, gereinigt.
- Man erhält: 2,0 g (7,1 mmol) 4-n-Butyl-6-chlor-3,4-dihydro-4-hydroxy-chinolin-2(1H)-on
als hellgelbes Öl (36 % d. Theorie) R_{F}-Wert = 0,47 (Kieselgelplatten; mob. Phase = n-Heptan/Ethylacetat = 1:1)

¹H-NMR (200 MHz, d₆-DMSO): = 0.73 (t, J = 7.5 Hz), 0.86 (s, 3H), 1.13 (s, 3H), 0.98- 1.35 ( 2 m, 6H), 1.36 - 1.79 (m, 2H), 5.11 (br s, 1H, OH-Gruppe), 7.81 (d, J = 8 Hz, 1H), 7.20 (dd, J = 8 und 2.5 Hz, 1H), 7.35 (d, J = 2.5 Hz, 1 Hz), 10.12 (br s, 1H)
MS: (M + H)⁺ = 282

### Beispiel 2

### 4-n-Butylen-6-chlor-3,4-dihydro-3,3-dimethyl-chinolin-2(1H)-on

1,4 g (4.97 mmol) 4-n-Butyl-6-chlor-3,4-dihydro-4-hydroxy-chinolin-2(1H)-on (Beispiel 1) werden in 50 ml absolutem Toluol gelöst und nach Zugabe von 100 mg p-Toluolsulfonsäure eine Stunde unter Rückfluß erhitzt.
Zur Aufarbeitung des Reaktionsgemisches wird nach Beendigung der Reaktion und Abkühlen auf Zimmertemperatur mit 200 ml Essigsäureethylester versetzt und die organische Phase mit 100 ml gesättigter wässriger Natriumbicarbonatlösung und zweimal mit jeweils 150 ml Wasser extrahiert. Nach Trocknung der organischen Phase mittels Natriumsulfat wird am Rotationsverdampfer unter reduziertem Druck eingeengt. Man erhält einen öligen hellgelben Rückstand, welcher bei Zugabe von n-Pentan kristallisiert.
- Ausbeute:: 0.87 g (3.3 mmol; 66 % d. Theorie),
farblose Kristalle vom Schmelzpunkt 144 °C; R_{F} = 0.58, mobile Phase: n-Heptan/Ethylacetat = 1/1

Das Reaktionsprodukt liegt lt. ¹H-NMR-Spektrum als ein E/Z-Diastereomerengemisch vor. Das E/Z-Verhältnis beträgt lt. HPLC (Säule: Nukleosil RP 18, 5m, 200 x 4.6 mm; Laufmittel: CH₃CN/Puffer = 40/60 mit einem Puffer von Wasser/Methanol/H₃PO₄/NEt₃ = 750/403/0.5; Fluß: 1ml/min) sowie ¹H-NMR-Spektrum 75/25.

### Trennung der E/Z Diastereomeren

600 mg des oben hergestellten Diastereomerengemisches werden über Sephadex (Typ LH-20, Fa. Fluka) mit Methanol als mobiler Phase chromatographiert:
Man erhält neben Mischfraktionen 320 mg E-4-n-Butylen-6-chlor-3,4-dihydro-3,3-dimethyl-chinolin-2(1H)-on vom Schmelzpunkt 157- 158 °C und 31 mg Z-4-n-Butylen-6-chlor-3,4-dihydro-3,3-dimethyl-chinolin-2(1H)-on vom Schmelzpunkt 170 - 171 °C, beide Fraktionen mit Reinheiten von jeweils größer 97 %.

### E-4-n-Butylen-6-chlor-3,4-dihydro-3,3-dimethyl-chinolin-2(1H)-on (73)

¹H-NMR (200 MHz, d₆-DMSO): = 0.87(t, J = 7.5 Hz, 3H), 1.18 (s, 6H), 1.44 (qt, J = 7.5 Hz, 2H), 2.25 (q, J = 7.5 Hz, 2 H), 5.73 (t, J = 7.5 Hz, 1 H), 6.93 (d, J = 10 Hz, 1H), 7.28 (s, 1 H), 7.33 (d, J = 10 Hz, 1 H), 10.28 (br s, 1 H)
MS: (M + H)⁺ = 264

### Z-4-n-Butylen-6-chlor-3,4-dihydro-3,3-dimethyl-chinolin-2(1H)-on (74)

¹H-NMR (200 MHz, d₆-DMSO): = 0.93 (t, J = 7.5 Hz, 3H), 1.36 (s, 6H), 1.48 (qt, J = 7.5 Hz, 2H), 2.41 (q, J = 7.5 Hz, 2 H), 5.88 (t, J = 7.5 Hz, 1 H), 6.86 (d, J = 10 Hz, 1H), 7.23 (dd, J = 2.5 und 10 Hz, 1 H), 7.42 (d, J = 2.5 Hz, 1 H), 10.32 (br s, 1 H)
MS: (M + H)⁺ = 264

### Beispiel 3

### E-4-n-Butylen-6-chlor-3,4-dihydro-3,3-dimethyl-chinolin-2(1H)-thion (75)

200 mg (0,76 mmol) E-4-n-Butylen-6-chlor-3,4-dihydro-3,3-dimethyl-chinolin-2(1H)-on (Beispiel 2) werden in 20 ml absolutem Toluol gelöst, mit 170 mg (0,42 mmol) Lawesson's Reagenz (2,4-Bis-(4-methoxyphenyl)-1,3-dithia-2,4-diphosphetan-2,4-disulfid) versetzt und das Reaktionsgemisch zwei Stunden auf 100 °C erhitzt.
Nach beendeter Reaktion wird das Lösungsmittel im Vakuum abgezogen und der Rückstand an Kieselgel chromatographiert. Mit n-Heptan/Essigsäureethylester, Mischungsverhältnis 2:1, als Elutionsmittel können 140 mg (66 %) des gewünschten Produktes isoliert werden (gelbliche Kristalle vom Schmelzpunkt 160 °C).
¹H-NMR (200 MHz, d₆-DMSO): = 0.87 (t, J = 7 Hz, 3H), 1.27 (s, 6H), 1.44 (qt, J = 7 Hz, 2H), 2.26 (q, J = 7 Hz, 2 H), 5.86 (t, J = 7 Hz, 1 H), 7.18 (d, J = 9.5 Hz, 1H), 7.33 - 7.40 (2 m, 2 H), 12.33 (br s, 1 H)
MS: (M + H)⁺ = 280

### Beispiel 4

### 4-n-Butyl-3,4-dihydro-3,3-dimethyl-4-hydroxy-6-methoxy-chinolin-2(1H)-on (V.23)

2g (9 mmol) 3,3-Dimethyl-6-methoxy-1,3-dihydrochinolin-2,4-dion (Beispiel III) werden in absolutem Tetrahydrofuran gelöst und bei einer Temperatur von -20 °C mit 13 ml einer 2 molaren Lösung von n-Butylmagnesiumchlorid in THF versetzt. Anschließend läßt man auf Zimmertemperatur kommen und rührt 2 Stunden nach.
Anschließend wird die Reaktionslösung auf 250 ml gesättigte wässrige Natriumchlorid-Lösung gegeben und das Gemisch mit 20-prozentiger wässr. Citronensäure-Lösung angesäuert (pH 3). Es wird mit 100 ml Essigsäureethylester dreimal ausgeschüttelt, die vereinigten organischen Phasen mittels Natriumsulfat getrocknet und unter reduziertem Druck am Rotationsverdampfer eingeengt. Das auf diese Weise erhaltene hellgelbe Öl wird mittels Chromatographie an Kieselgel (mobile Phase n-Heptan/Essigsäureethylester = 2/1) gereinigt.
- Man erhält: 1.2 g (48 % d. Theorie),
farbloses Öl

¹H-NMR (200 MHz, d₆-DMSO): 0.71 (t, J = 7.5 Hz, 3H), 0.85 (s, 3 H), 0.97 - 1.69 (3 m, 6 H), 1.10 (s, 3 H), 3.71 (s, 3 H), 4.92 (br s, 1 OH), 6.72 (2 ps s, 2 H), 6.95 (ps s, 1 H), 9.78 (br s, 1 H)
MS: (M + H)⁺ = 278

### Beispiel 5

### E/Z-4-n-Butylen-3,4-dihydro-3,3-dimethyl-6-methoxy-chinolin-2(1H)-on (41)

600 mg (2 mmol) 4-n-Butyl-3,4-dihydro-3,3-dimethyl-4-hydroxy-6-methoxychinolin-2(1H)-on (Beispiel 4) werden in 40 ml absolutem Toluol gelöst, mit einer Spatelspitze p-Toluolsulfonsäure versetzt und das Gemisch 3 Stunden auf 100 °C erhitzt. Nach Abkühlen des Reaktionsgemisches auf Zimmertemperatur wird die organische Phase über Natriumsulfat getrocknet und das Lösungsmittel unter reduziertem Druck am Rotationsverdampfer entfernt. Das Reaktionsprodukt kristallisiert beim Entfernen des Lösungsmittels aus.
- Ausbeute: 0.39 g (73 % d.Theorie),
Schmelzpunkt 121- 122°C
MS: (M + H)⁺ = 260
lt ¹H-NMR (200 MHz, d₆-DMSO) besteht das Reaktionsprodukt aus einem E/Z-Diastereomerengemisch. Das E/Z-Diasteremerenverhältnis beträgt ca. 10:1. Die Daten der Hauptkomponente ergeben sich zu:
= 0.89 (t, J = 7.5, 3H), 1.16 (s, 3 H), 1.45 (tq, J = 7.5 Hz, 2 H), 2.29 (dt, J = 7.5 Hz, 2 H), 3.73 (s, 3 H), 5.64 (t, J = 7.5 Hz, 1 H), 6.84 (br s, 3 H), 9.97 (br s, 1 H).

### Beispiel 6

### E/Z-4-n-Butylen-3,4-dihydro-3,3-dimethyl-6-methoxy-chinolin-2(1H)-thion (42) und E-4-n-Butylen-3,4-dihydro-3,3-dimethyl-6-methoxy-chinolin-2(1H)-thion (79)

0.23 g (0.89 mmol) E/Z-4-n-Butylen-3,4-dihydro-3,3-dimethyl-6-methoxychinolin-2(1H)-on (s. Beispiel 5) werden in 12 ml absolutem Toluol gelöst und mit 0.22 g Lawesson's Reagenz (2,4-Bis-(4-methoxyphenyl)-1,3-dithia-2,4-diphosphetan-2,4-disulfid) versetzt. Das Reaktionsgemisch wird drei Stunden auf 100 °C erhitzt.
Nach beendeter Reaktion wird das Lösungsmittel im Vakuum destilliert und der Rückstand an Kieselgel chromatographiert. Mit n-Heptan/Essigsäureethylester, Mischungsverhältnis 3:1, als Elutionsmittel können 239 mg (94 %) der Zielverbindung isoliert werden (farblose Kristalle vom Schmelzpunkt 133-134 °C).
MS: (M + H)⁺ = 276

It. ¹H-NMR-Spektrum sowie HPLC-Untersuchung liegt ein 70:30 E/Z-Diastereomerengemisch vor.

Zur Isolierung des E-Diastereomeren werden 120 mg der E/Z-Diastereomerenmisch über Sephadex (Typ LH-20) mittels Methanol als mobiler Phase chromatographiert. Es lassen sich 70 mg E-4-n-Butylen-3,4-dihydro-3,3-dimethyl-6-methoxy-chinolin-2(1H)-thion in 97%iger Reinheit (HPLC-Analyse) isolieren (Schmelzpunkt: 135 - 136 °C).

### E-4-n-Butylen-3,4-dihydro-3,3-dimethyl-6-methoxy-chinolin-2(1H)-thion (79)

¹H-NMR (200 MHz, d₆-DMSO): 0.88 (t, J = 7 Hz, 3 H), 1.26 (s, 3 H), 1.43 (tq, J = 7 Hz, 2 H), 2.27 (dt, J = 7 Hz, 2 H), 3.76 (s, 3 H), 5.81 (t, J = 7 Hz, 1 H), 6.85 (m, 1 H), 6.88 (dd, J = 2 und 8.5 Hz, 1 H), 7.08 (d, J = 8.5 Hz, 1 H), 12.14 (br s, 1 H)
MS: (M + H)⁺ = 276

### Beispiel 7

### 4-n-Butyl-3,3-cyclopentyliden-3,4-dihydro-4-hydroxy-chinolin-2(1H)-on (V.5)

4.3 g (0,02 mol) 3,3-Cyclopentyliden-chinolin-2,4(1H,3H)-dion (hergestellt gemäß Beispiel I, jedoch unter Verwendung von Cyclopentancarbonsäureethylester anstelle von Isobuttersäure-ethylester) werden in 100 absolutem Tetrahydrofuran gelöst und bei einer Temperatur von -30 °C mit 30 ml n-Butylmagnesiumchlorid (2m Lösung in THF) versetzt. Nach Beendigung der Zugabe wird drei Stunden bei 25 °C nachgerührt. Anschließend wird die Reaktionslösung auf 200 ml gesättigte wässrige Natriumchlorid-Lösung gegeben und das Gemisch mit 10-prozentiger wässr. Citronensäure-Lösung angesäuert (pH 3). Es wird mit 150 ml Essigsäureethylester dreimal ausgeschüttelt, die vereinigten organischen Phasen mittels Natriumsulfat getrocknet und unter reduziertem Druck am Rotationsverdampfer eingeengt. Das auf diese Weise erhaltene hellgelbe Öl wird mittels Chromatographie an Kieselgel, mobile Phase n-Heptan/Essigsäureethylester = 2/1, gereinigt. Man erhält 2,62g (48%) des gewünschten Reaktionsproduktes (Schmelzpunkt 95 - 97°C).

¹H-NMR (200 MHz, d₆-DMSO): = 0.7 (t, J = 7.5 Hz, 3H), 0.96 - 2.20 (m, 14 H), 4.93 (br s, 1 H), 6.79 (dd, J = 7 und 1 Hz, 1 H), 6.95 (dt, J = zweimal 7 und einmal 1 Hz, 1 H), 7.14 (dt, J = zweimal 7 und einmal 1 Hz, 1 H), 7.37 (dd, J = 7 und 1 Hz, 1 H), 9.85 (br s, 1 H)
MS: (M + H)⁺ = 274

### Beispiel 8

### E/Z-4-n-Butylen-3,3-cyclopentyliden-3,4-dihydro-chinolin-2(1H)-on (6)

2 g (7.3 mmol) 4-n-Butyl-3,3-cyclopentyliden-3,4-dihydro-4-hydroxy-chinolin-2(1H)-on (s. Versuch 7) werden in 100 ml absolutem Toluol gelöst und zusammen mit einer Spatelspitze p-Toluolsulfonsäure zwei Stunden unter Rückfluß erhitzt. Nach Beendigung der Reaktion wird das Lösungsmittel unter reduziertem Druck am Rotationsverdampfer eingeengt und der Rückstand aus n-Pentan umkristallisiert.
Ausbeute: 1.75 g (94 %); Schmelzpunkt 115- 117 °C
MS: (M + H)⁺ = 256

### Beispiel 9

E/Z-4-n-Butylen-3,3-cyclopentyliden-3,4-dihydro-chinolin-2(1H)-thion (8) und E-4-n-Butylen-3,3-cyclopentyliden-3,4-dihydro-chinolin-2(1H)-thion (80) 1 g (3.9 mmol) E/Z-4-n-Butylen-3,3-cyclopentyliden-3,4-dihydro-chinolin-2(1H)-on (siehe Beispiel 8) werden in 100 ml abs Toluol gelöst und mit 0.89 g Lawesson's Reagenz (2,4-Bis-(4-methoxyphenyl)-1,3-dithia-2,4-diphosphetan-2,4-disulfid) versetzt. Das Reaktionsgemisch wird drei Stunden unter Rückfluß erhitzt.
Nach beendeter Reaktion wird das Lösungsmittel im Vakuum abgezogen und der Rückstand an Kieselgel chromatographiert. Mit n-Heptan/Essigsäureethylester, Mischungsverhältnis 3:1, als Elutionsmittel und anschließender Kristallisation aus n-Pentan kann das gewünschte Reaktionsprodukt isoliert werden.
Ausbeute: 0.51g (48 %); Schmelzpunkt: 81 - 83 °C
lt. ¹H-NMR-Spektrum sowie HPLC-Untersuchung liegt ein 82 : 18 E/Z-Diastereomeren-gemisch vor.

Zur Isolierung des E-Diastereomeren werden 300 mg der E/Z-Diastereomerenmisches über Sephadex (Typ LH-20) mittels Methanol als mobiler Phase chromatographiert. Man erhält neben Mischfraktionen beider Diastereomeren 150 mg E-4-n-Butylen-3,3-cyclopentyliden-3,4-dihydro-chinolin-2(1H)-thion, Schmelzpunkt 80-81°C.

¹H-NMR (200 MHz, d₆-DMSO): = 0.86 (t, J = 7 Hz,3 H), 1.43 (tq, J = 7 Hz, 2 H), 1.45 - 1.85 (3 m, 6 H), 2.06 - 2.18 (m, 2 H), 2.23 (td, J = 7 Hz, 2 H), 5.74 (t, J = 7 Hz, 1 H), 7.08 - 7.19 (m, 2 H), 7.25 - 7.36 (m, 2 H), 12.18 (br s, 1 H)
MS: (M + H)⁺ = 272

### Beispiel 10

### 3,4-Dihydro-3,3-dimethyl-4-hydroxy-4-(3-methyl-3-propen-1-yl)-chinolin-2(1H)-on (V.38)

Aus 1.1 g (45 mmol) Magnesiumspänen und 2.34 ml (23.8 mmol) 3-Chlor-2-methyl-1-propen sowie 30 ml absolutem THF als Lösungsmittel wird eine entsprechende Lösung von 2-Methyl-2-propenylmagnesiumchlorid in THF hergestellt. Zu dieser Lösung gibt man bei 25 °C 2.2 g (11.9 mmol) 3,3-Dimethyl-6-methoxy-1,3-dihydrochinolin-2,4-dion (Verbindung aus Beispiel III), welche voher in 25 ml abs. THF gelöst wurde.
Zur Aufarbeitung wird anschließend die Reaktionslösung auf 100 ml gesättigte wässrige Natriumchlorid-Lösung gegeben und das Gemisch mit 20 %iger wässr. Citronensäure-Lösung angesäuert (pH 3). Anschließend wird dreimal mit jeweils 100 ml Essigsäureethylester ausgeschüttelt, die vereinigte organische Phase mittels Natriumsulfat getrocknet und unter reduziertem Druck am Rotationsverdampfer eingeengt. Das auf diese Weise erhaltene hellgelbe Rohprodukt wird mittels Chromatographie an Kieselgel; mobile Phase n-Heptan/Essigsäureethylester = 2/1, gereinigt.
- Man erhält: 1.27 g der gewünschten Verbindung
als ein hellgelbes Öl (Ausbeute 44 %).
MS: (M + H)⁺ = 278

### Beispiel 11

### 3,4-Dihydro-3,3-dimethyl-4-(3-methyl-3-propenylen)-chinolin-2(1H)-on (82)

1 g (4 mmol) 3,4-Dihydro-3,3-dimethyl-4-hydroxy-4-(3-methyl-3-propen-1-yl)-chinolin-2(1H)-on (Beispiel 10) werden in 60 ml absolutem Toluol gelöst und mit 20 mg p-Toluolsulfonsäure versetzt. Das Reaktionsgemisch wird drei Stunden auf 100 °C erhitzt und der Verlauf der Umsetzung dünnschichtchromatographisch verfolgt.
Nachdem das Reaktionsgemisch auf Zimmertemperatur abgekühlt ist wird das Reaktionsgemisch mit 100 ml gesättigter Natriumbicarbonatlösung und dreimal mit jeweils 100 ml Wasser extrahiert. Nach Trocknen der organischen Phase über Magnesiumsulfat mit Einengen unter reduziertem Druck am Rotationsverdampfer wird der verbleibende ölige Rückstand mit n-Pentan verrührt. Das Reaktionsprodukt, 3,4-Dihydro-3,3-dimethyl-4-hydroxy-4-(3-methyl-3-propen-1-yl)-chinolin-2(1H)-on, kristallisiert nach einiger Zeit in Form farbloser Kristalle aus.
- Ausbeute:: 0.81 g (89 % d. Theorie); Schmelzpunkt: 151- 153 °C

¹H-NMR (200 MHz, d₆-DMSO): = 1.19 (2 s, 6 H), 1.73 (2, 3H), 4.85 (br s, 1H), 4.98 (br s, 1H), 6.19 (br s, 1H), 6.89 (d, J = 7.5 Hz, 1 H), 6.93 (ddd, J = 7.5 (2 mal) und 1 Hz), 7.21 (ddd, J = 1 und 7.5 Hz (2 mal), 7.34 (d, J = 7.5 Hz, 1 H), 10.96 (br s, 1 H)
MS: (M + H)⁺ = 228

### Beispiel 12

### 3,4-Dihydro-3,3-dimethyl-4-(3-methyl-3-propenylen)-chinolin-2(1H)-thion (83)

0.6 g (2.6 mmol) 3,4-Dihydro-3,3-dimethyl-4-(3-methyl-3-propenylen)-chinolin-2(1H)-on (s. Beispiel 11) werden in 20 ml abs. Toluol gelöst und mit 0.64 g Lawesson's Reagenz (2,4-Bis-(4-methoxyphenyl)-1,3-dithia-2,4-diphosphetan-2,4-disulfid) versetzt. Das Reaktionsgemisch wird 8 Stunden unter Rückfluß erhitzt.
Zur Aufarbeitung wird das Lösungsmittel am Rotationsverdampfer unter reduziertem Druck entfernt und das Rohprodukt mittels Chromatographie an Kieselgel (mobile Phase: n-Heptann-Heptan/Essigsäureethylester = 3/1) gereinigt. Das Reaktionsprodukt kristalliert anschließend beim Entfernen des Lösungsmittels am Rotationsverdampfer unter reduziertem Druck aus.
- Ausbeute:: 320 mg (51 % d. Theorie); Schmelzpunkt 140 - 142°C

¹H-NMR (200 MHz, d₆-DMSO): = 1.31 (2 s, 6 H), 1.72 (s, 3H), 4.83 (br s, 1H), 4.98 (br s, 1H), 6.33 (br s, 1H), 7.08 (ddd, J = 14, 8 und 1 Hz, 1H), 7.26 (m, 1 H), 7.38 (d, J = 8 Hz, 1 H), 12.25 (br s, 1 H)
MS: (M + H)⁺ = 244

### Beispiel 13

### E/Z-4-n-Butylen-6-chlor-3,4-dihydro-1,3,3-trimethyl-chinolin-2(1H)-on (91)

264 mg 4-n-Butylen-6-chlor-3,4-dihydro-3,3-dimethyl-chinolin-2(1H)-on (s. Beispiel 2) werden in 20 ml absolutem N,N-Dimethylformamid gelöst und bei einer Temperatur von 25°C unter Rühren mit 53 mg Natriumhydrid (50 %ige Suspension in Öl) versetzt. Nach Beendigung der Wasserstoffentwicklung wird das Reaktionsgemisch mit 280 mg Methyliodid versetzt und anschließend eine Stunde bei 25 °C gerührt. Es scheidet sich ein farbloser Niederschlag ab.
Zur Aufarbeitung wird das Lösungsmittel am Rotationsverdampfer im Ölpumpenvakuum abdestilliert und der verbleibende Rückstand mit Wasser/Ethylacetat extrahiert. Die vereinigte organische Phase wird anschließend über Na₂SO₄ getrocknet und das Extraktionsmittel am Rotationsverdampfer unter reduziertem Druck entfernt. Man erhält einen hellgelben öligen Rückstand, der durch Chromatographie am Kieselgel mit n-Heptan/Ethylacetat = 3/1 als mobiler Phase gereinigt wird.
- Ausbeute:: 270 mg (97 % d. Th.),
hellgelbes Öl

¹H-NMR (200 MHz, d₆-DMSO): = 0.86 (t, J = 7.5Hz, 3H), 1.14 (s, 6H), 1.44 (tq, J = 7.5Hz, 2H), 2.25 (dt, J = 7.5Hz, 2H), 3.25 (s, 3H), 5.68 (t, J = 7.5Hz, 1H), 7.18 (d, J = 9Hz, 1H), 7.28-7.44 (m, 2H)
MS: (M + H)⁺ = 278

### Beispiel 14

### E/Z-4-n-Butylen-6-chlor-3,4-dihydro-1,3,3-trimethyl-chinolin-2(1H)-thion (87)

150 mg E/Z-4-n-Butylen-6-chlor-3,4-dihydro-1,3,3-trimethyl-chinolin-2(1H)-on (s. Beispiel 13) werden gem. Beispiel 12 mit Lawesson's Reagenz umgesetzt. Die chromatographische Reinigung des erhaltenen Rohproduktes erfolgt mittels Säulenchromatographie an Kieselgel mit n-Heptan/Ethylacetat = 7/1 als mobiler Phase.
- Ausbeute:: 110 mg, hellgelbes Öl
¹H-NMR (200 MHz, d₆-DMSO): = 0.88 (t, J = 7.5Hz, 3H), 1.25 (s, 6H), 2.45 (tq, J = 7.5Hz, 2H), 2.23 (dt, J = 7.5Hz, 2H), 3.80 (s, 3H), 5.83 (t, J = 7.5Hz, 1H), 7.27-7.55 (m, 3H)
MS: (M + H)⁺ = 294

### Beispiel 15

### E/Z-4-n-Butylen-6-chlor-3,4-dihydro-1,3,3-trimethyl-chinolin-2(1H)-on-2-oxim (90)

1.5 g (5.4 mmol) E/Z-4-n-Butylen-6-chlor-3,4-dihydro-3,3-dimethyl-chinolin-2(1H)-on (s. Beispiel 2) werden in 40 ml absolutem Ethanol gelöst und unter Rühren mit 751 mg (10.8 mmol) Hydroxylaminhydrochlorid sowie 1.5 ml (10.8 mmol) Triethylamin versetzt. Das Reaktionsgemisch wird 48 Stunden bei 25 °C gerührt.
Zur Aufarbeitung wird das Reaktionsgemisch am Rotationsverdampfer unter reduziertem Druck eingeengt und der verbleibende Rückstand mit Essigsäureethylester/Wasser extrahiert. Die vereinigte organische Phase wird mit Na₂SO₄ getrocknet und das Extraktionsmittel am Rotationsverdampfer unter reduziertem Druck entfernt. Der verbleibende Rückstand kristallisiert beim Versetzen mit n-Pentan aus.
- Ausbeute:: 1.43 g (95% d. Theorie),
farblose Kristalle vom Schmelzpunkt 163 - 165 °C

¹H-NMR (200 MHz, d₆-DMSO): = 0.89 (t, J =7.5Hz, 3H), 1.19 (s, 6H), 1.43 (tq, J = 7.5Hz, 2H), 2.25(dt, J = 7.5Hz, 2H), 5.51 (t, J =7.5Hz, 1H), 7.05-7.21 (m, 3H), 9.03 (s, 1H), 9.74 (s, 1H)
MS: (M + H)⁺ = 279

### Beispiel 16

### E/Z-4-Butylen-3,4-dihydro-3,3-dimethyl-6-hydroxy-chinolin-2(1H)-on (106)

1.5 g (5.8 mmol) E/Z-4-Butylen-3,4-dihydro-3,3-dimethyl-6-methoxy-chinolin-2(1H)-on (Beispiel 5) werden in 80 ml 1,2 Dichlorethan gelöst und bei 25°C mit 5 ml Trimethylsilyliodid versetzt. Anschließend wird 12 Stunden unter Rückfluß erhitzt.
Zur Aufarbeitung wird das Reaktionsgemisch unter Eiskühlung mit 100 ml Methanol versetzt und das Lösungsmittel am Rotationsverdampfer unter reduziertem Druck entfernt. Der verbleibende Rückstand wird mittels Chromatographie an Kieselgel (Elutionsmittel:Ethylacetat/n-Heptan im Mischungsverhältnis 1 zu 3) gereinigt. Zur Feinreinigung des Reaktionsproduktes eignet sich eine Sephadex-Säule (Typ LH-20, Fa. Fluka) mit Methanol mobiler Phase.
- Ausbeute:: 220 mg (16%); Schmelzpunkt 162-163°C

¹H-NMR (200 MHz, d₆-DMSO): δ= 0.89 (t,3H), 1.13 (s,6H), 1.44 (tq,2H), 2.26 (dt,2H), 5.60 (t,1H), 6.8-6.58 (3m,3H), 9.14 (br,s,10H), 10.83 (s,1NH)
MS: (M + H)⁺ = 246

### Beispiel 17

### 4-E-n-Butylen-1-oxycarbonylmethyl-3,4-dihydro-6,7-dimethoxy-3,3-dimethylchinolin-2(1H)-on (123)

0.35 g (0,97 mmol) 4-E-n-Butylen-3,4-dihydro-6,7-dimethoxy-3,3-dimethyl-1-methoxycarbonylmethyl-chinolin-2(1H)-on (Verbindung 122, Herstellung vergleiche Tabelle 3 und 4) werden in 20 ml Ethanol und mit 0.8 g NaOH-Plätzchen, welche zuvor in 20 ml Wasser gelöst waren, versetzt. Das Reaktionsgemisch wird 3 Stunden unter Rückfluß erhitzt.
Zur Aufarbeitung wird das Reaktionsgemisch am Rotationsverdampfer unter reduziertem Druck auf 1/3 des Volumens eingeengt und der Rückstand mit 2 n wässrige Salzsäure angesäuert. Nach Extraktion mittels Ethylacetat wird die organische Phase über Natriumsulfat getrocknet und das Lösungsmittel am Rotationsverdampfer abdestilliert. Das Reaktionsprodukt kristallisiert nach Zusatz von 10 ml n-Pentan in Form farbloser Kristalle aus.
- Ausbeute:: 0.28 mg (83% d.Th.), Schmelzpunkt 143-144°C

¹H-NMR (200 MHz, d₆-DMSO): δ= 0.89 (t,3H), 1.16 (s,6H), 1.46 (qt,2H), 2.31 (dt,2H), 3.75 (s,3H), 3.81 (s,3H), 4.56 (s,2H), 5.6 (t,1H), 6.61 (s,1H), 6.88 (s,1H), 12.76 (br,s,1H)
MS: (M + H)⁺ = 348

### Beispiel 18

### 4-Cyclopentyl-3,3-dimethyl-3,4-dihydro-4-hydroxy-chinolin-2(1H)-on

Eine Lösung von 0.18 mol Cyclopentyllithium in n-Pentan wird aus Lithiumpulver und Cyclopentylchlorid hergestellt. Diese Lösung wird bei einer Temperatur von -70°C in eine Lösung von 4.73 g (25 mmol) 3,3-Dimethyl-1,3-dihydrochinolin-2,4-dion, synthetisiert gemäß Beispiel I jedoch unter Verwendung von Isatinsäureanhydrid und Isobuttersäureethylester als Ausgangskomponenten, gelöst in 200 ml abs. Tetrahydrofuran, gegeben und das Reaktionsgemisch nach einstündigem Rühren bei dieser Temperatur auf 0°C erwärmt.
Zur Aufarbeitung wird mit 100 ml 20%iger wässriger Zitronensäurelösung versetzt, das Reaktionsgemisch auf Wasser gegeben und mit Ethylacetat extrahiert. Nach Trocknung mittels Natriumsulfat wird das Extraktionsmittel am Rotationsverdampfer unter reduziertem Druck abdestilliert. Das als Rohprodukt anfallende hellgelbe Öl wird mittels Chromatographie an Kieselgel (mobile Phase: n-Heptan/Ethylacetat = 2/1) gereinigt. Das Reaktionsprodukt iwrd anschließend aus n-Pentan umkristalllisiert.
- Ausbeute:: 4.95 mg (76% d.Th.), farblose Kristalle vom Schmelzpunkt 188-189°C

¹H-NMR (200 MHz, d₆-DMSO): δ = 0.60-0.78 (m,1H), 0.93 (s,3H), 1.02-1.50 (m,5H), 1.17 (s,3H), 1.66-181 (m,1H), 2.21 -2.05 (m,1H), 4.84 (s,10H), 6.78 (d,1H), 6.97 (t,1H), 7.16 (dt,1H), 7.40 (dt, 1H), 9.91 (s,1NH)
MS: (M + H)⁺ = 260

(Zur Freisetzung der OH-Gruppe mittels Trimethylsilyliodid siehe z.B. M.B. Jung und M.A. Lyster, J. Org. Chem. 42, 3764 (1977)).

Die folgende Tabelle 3 gibt eine Übersicht der synthetisierten Verbindungen. Alle Derivate wurden mittels ¹H-NMR-Spektrum, Massenspektrum sowie ihren jeweiligen Schmelzpunkt charakterisiert. Bei einer Vielzahl von Verbindungen handelt es sich um E/Z-Diastereomerengemische, welche in einigen Fällen mittels Chromatopgraphie an Sephadex (Typ LH-20, mobile Phase Methanol; siehe Beispiel 2) getrennt wurden. Diastereomerengemische sind in der Tabelle durch eine Schlangenlinie der betreffenden Bindung charakterisiert. Im Allgemeinen liegt dabei die E-Verbindung im Überschuß vor.

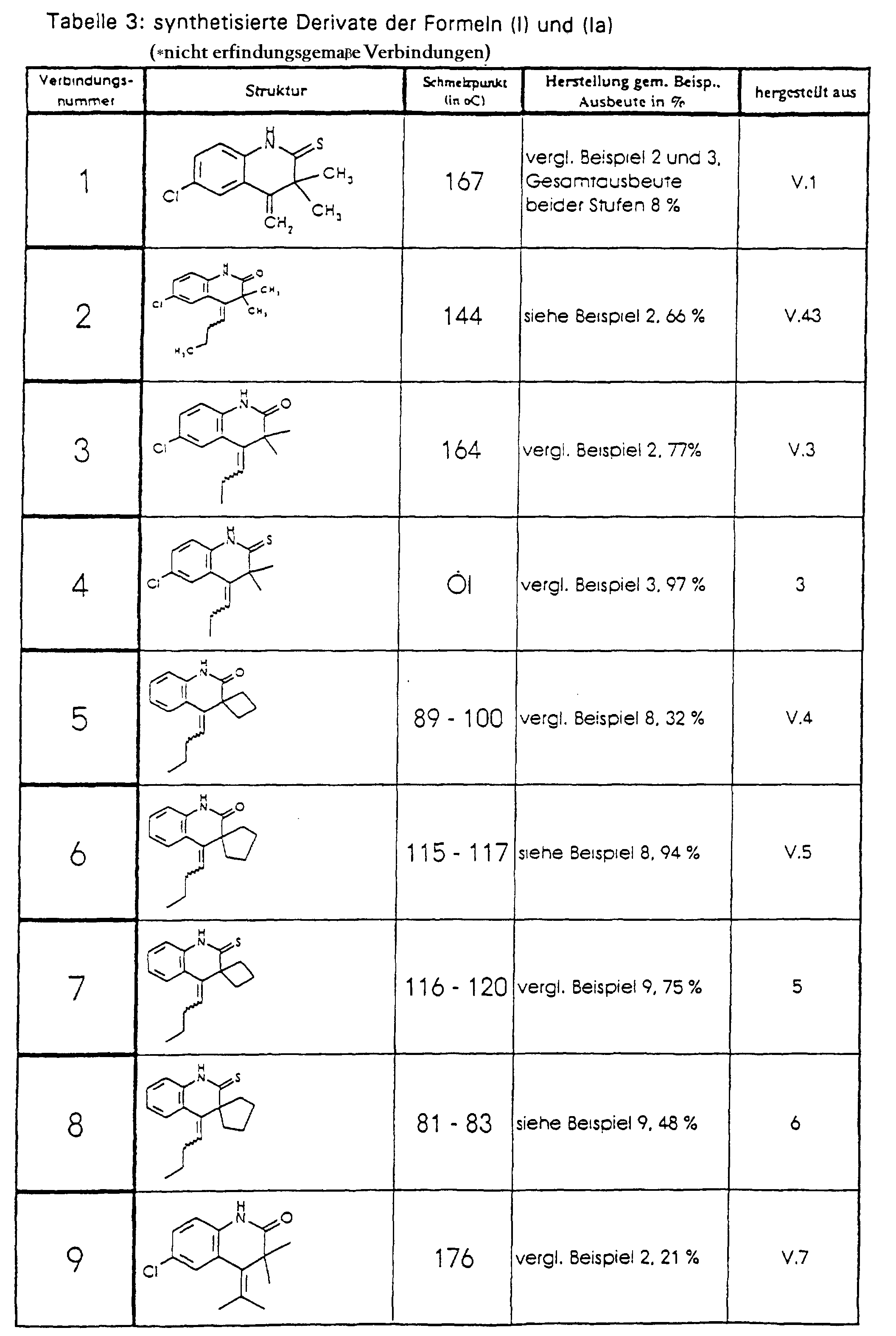

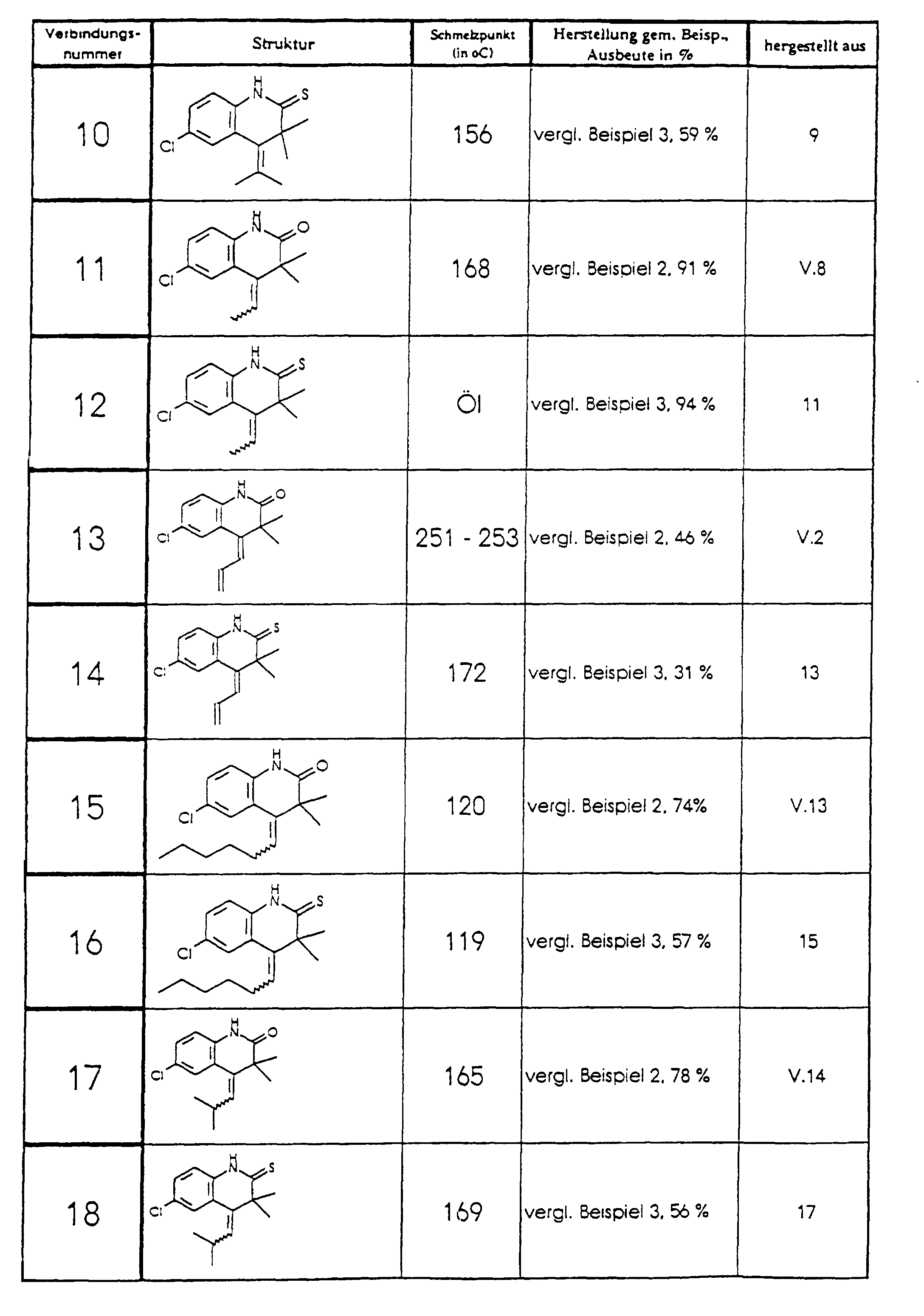

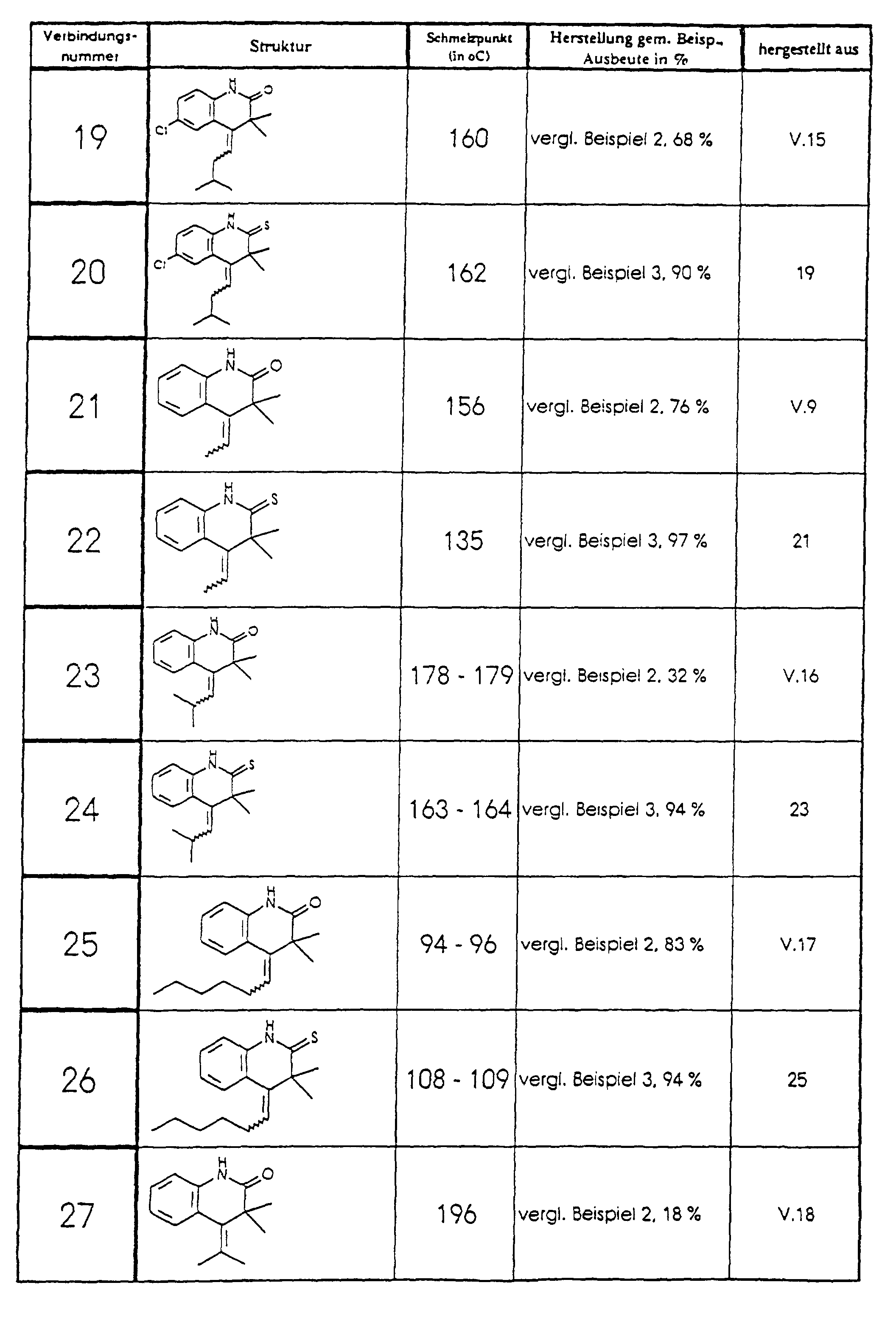

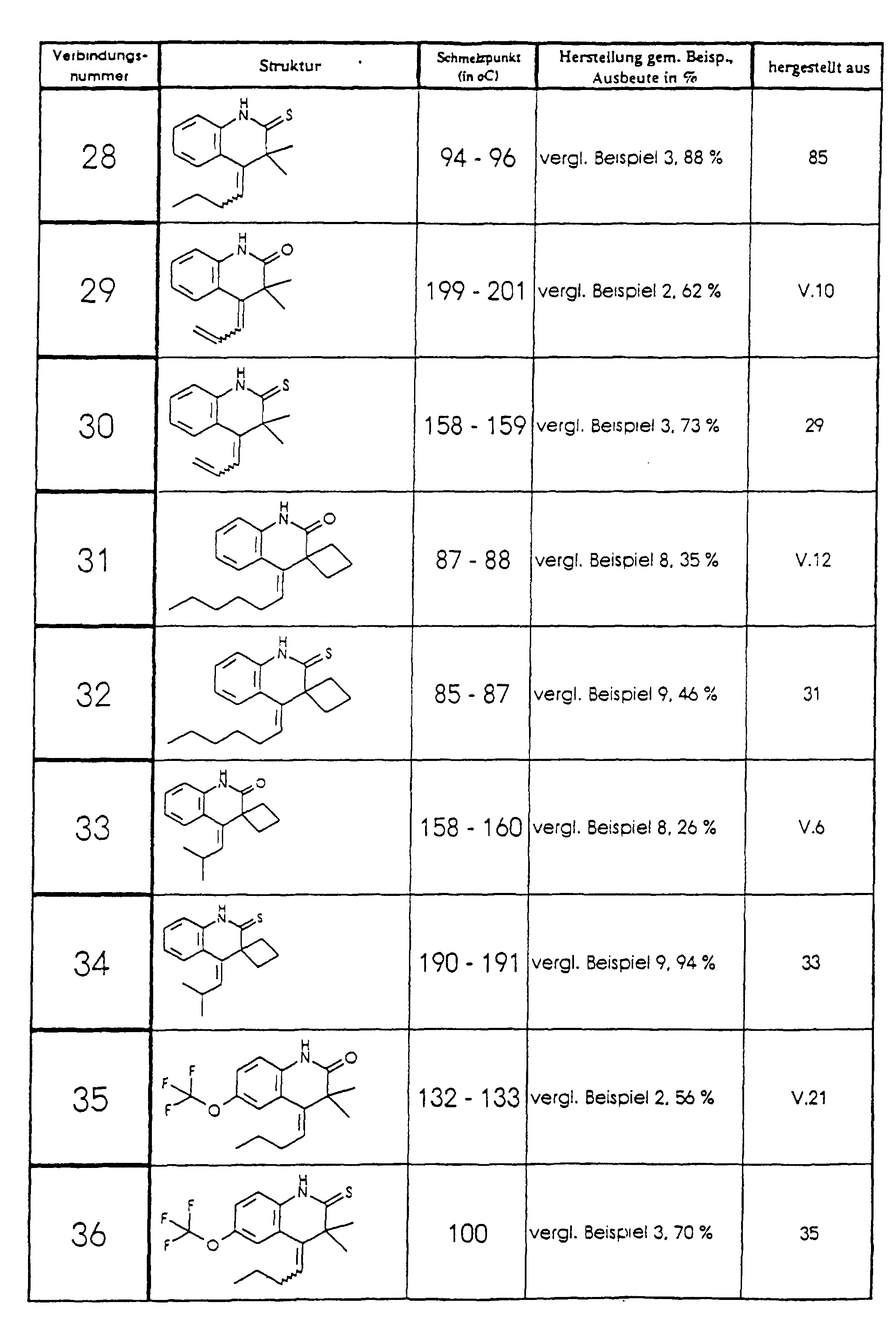

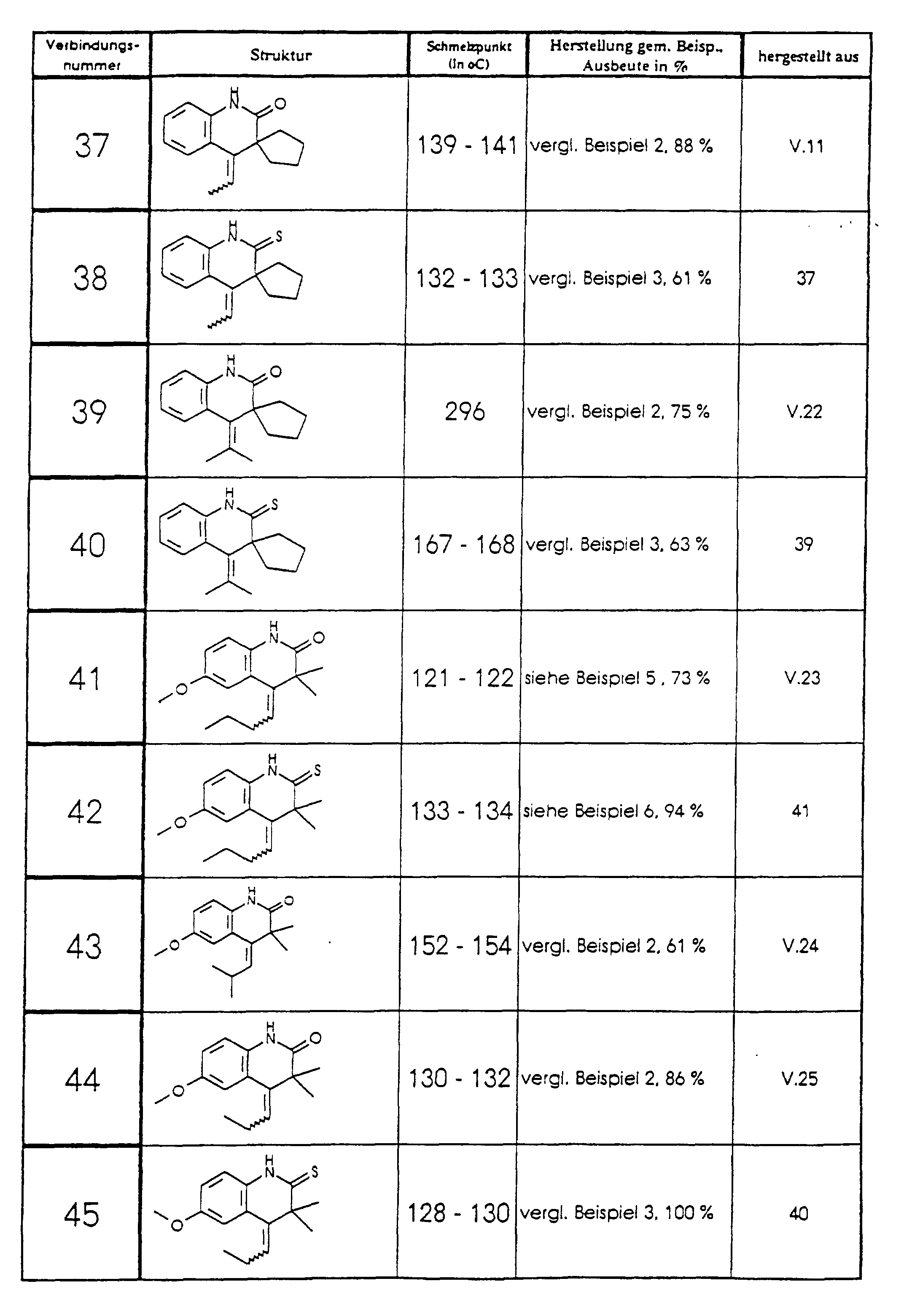

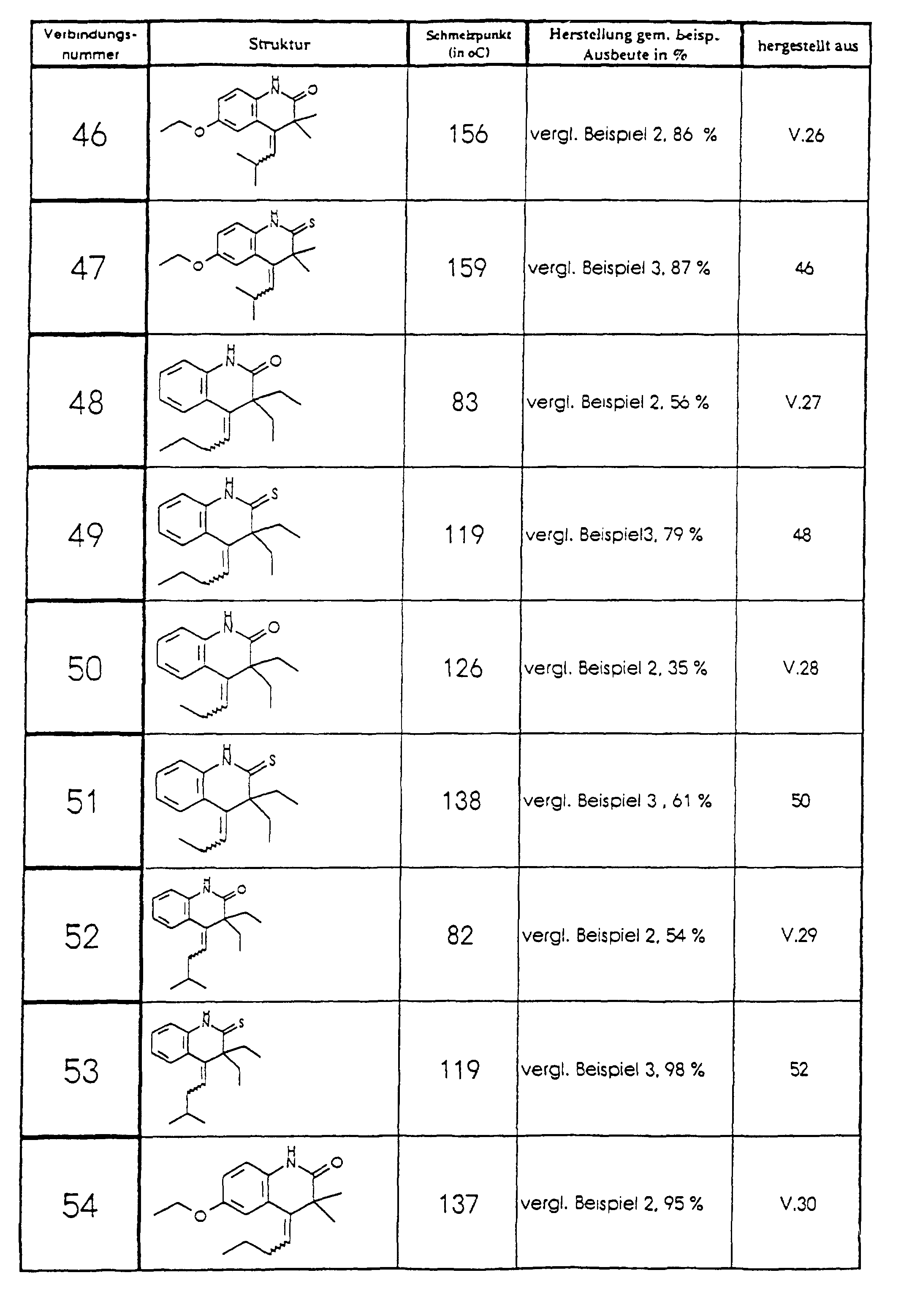

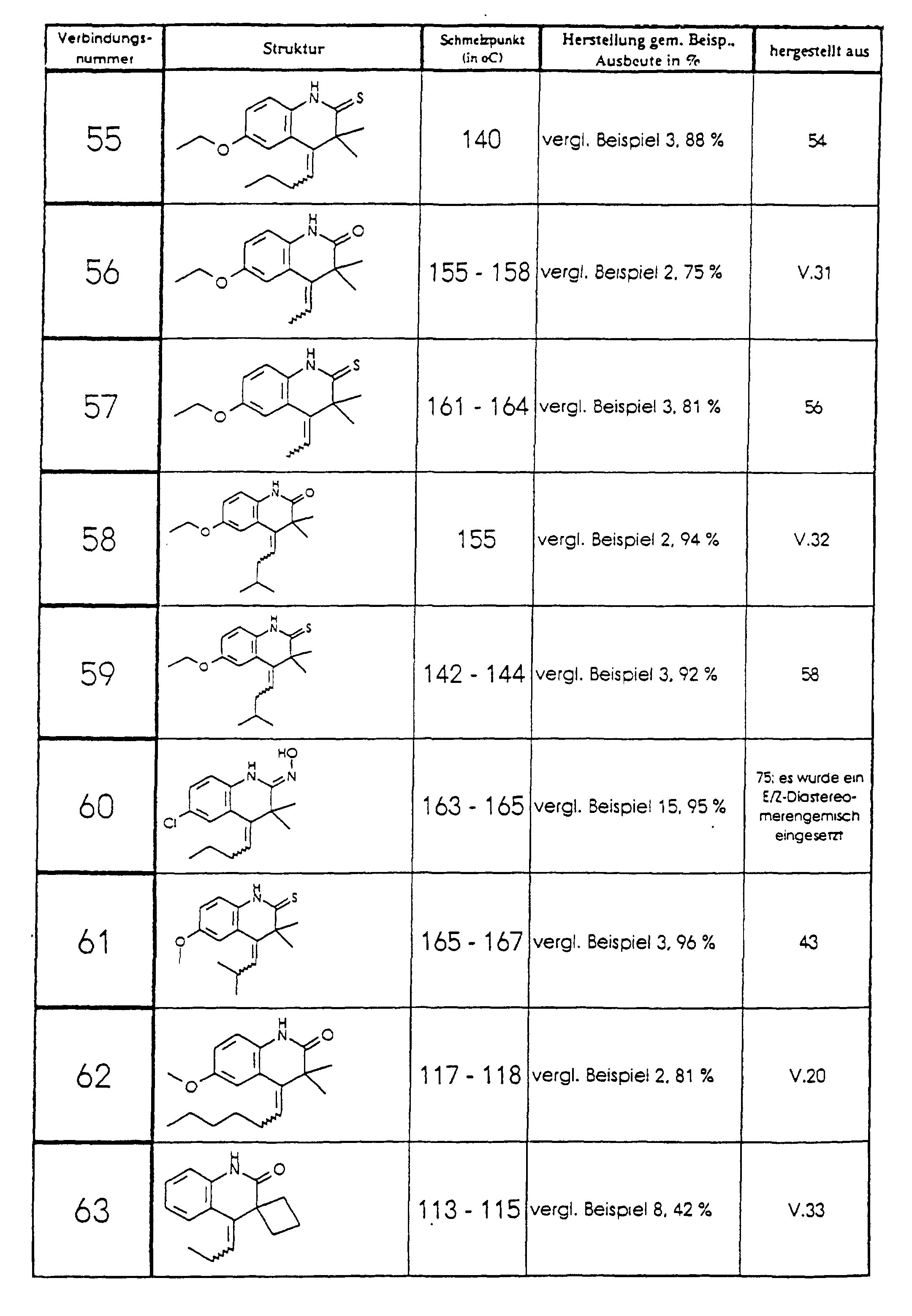

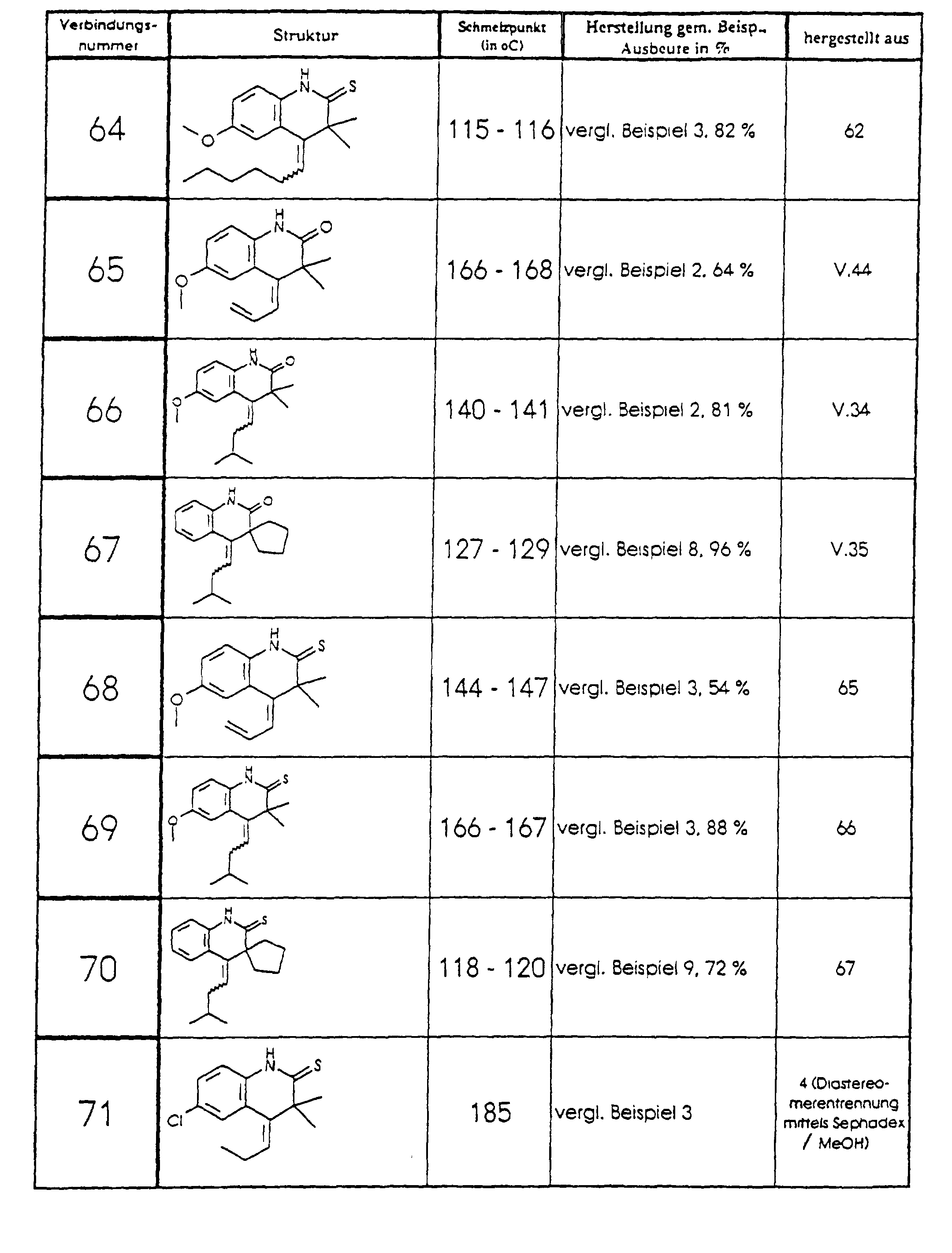

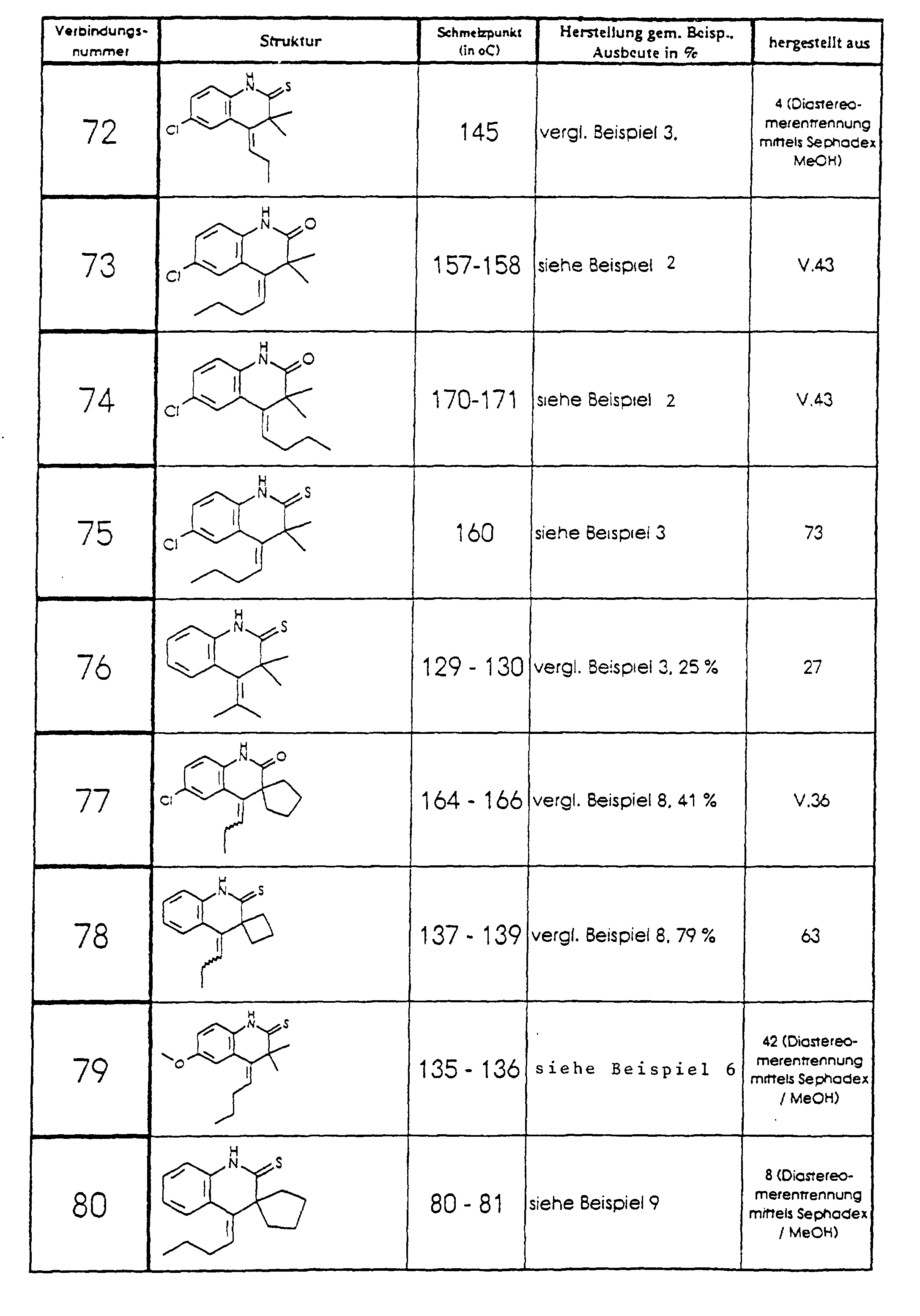

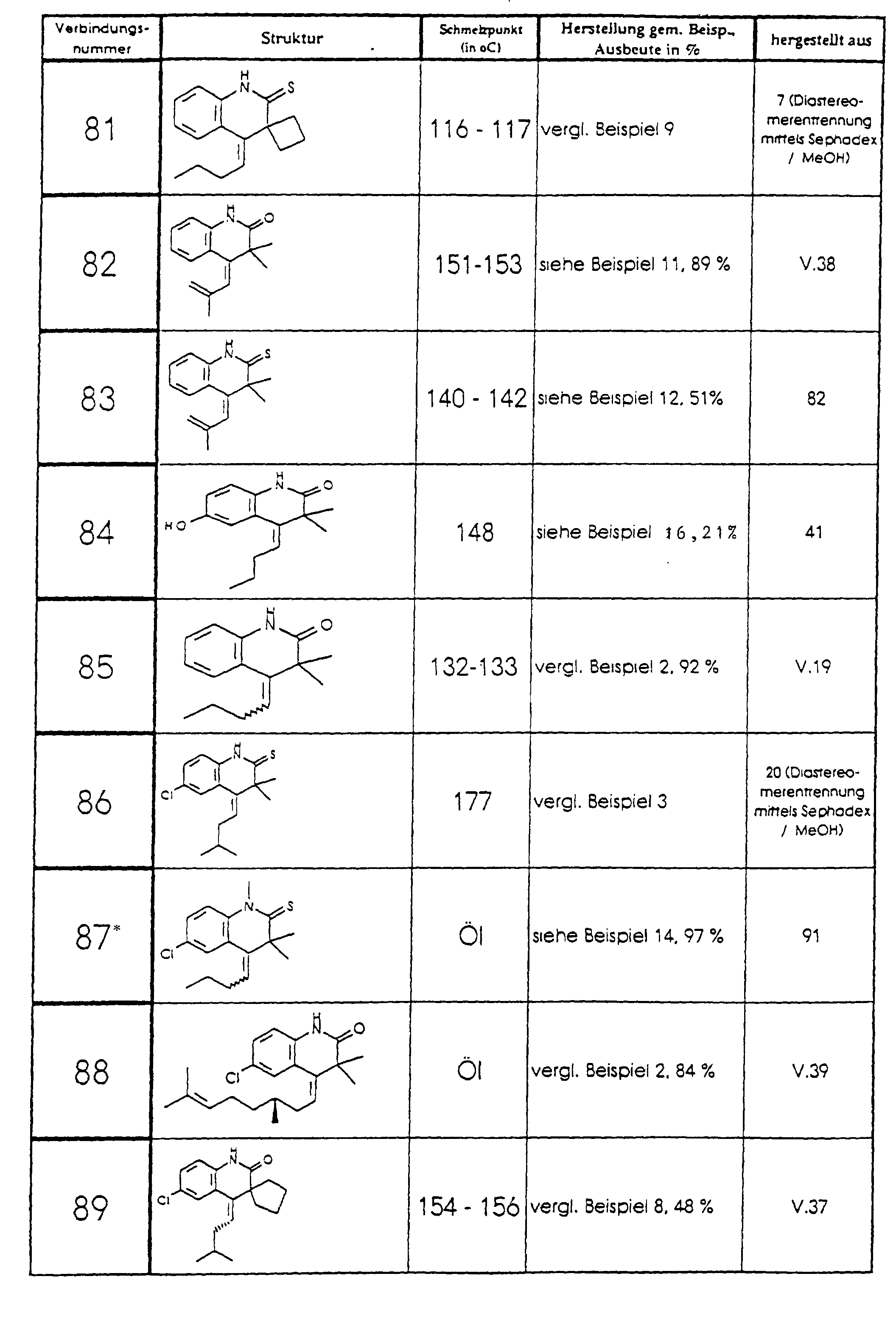

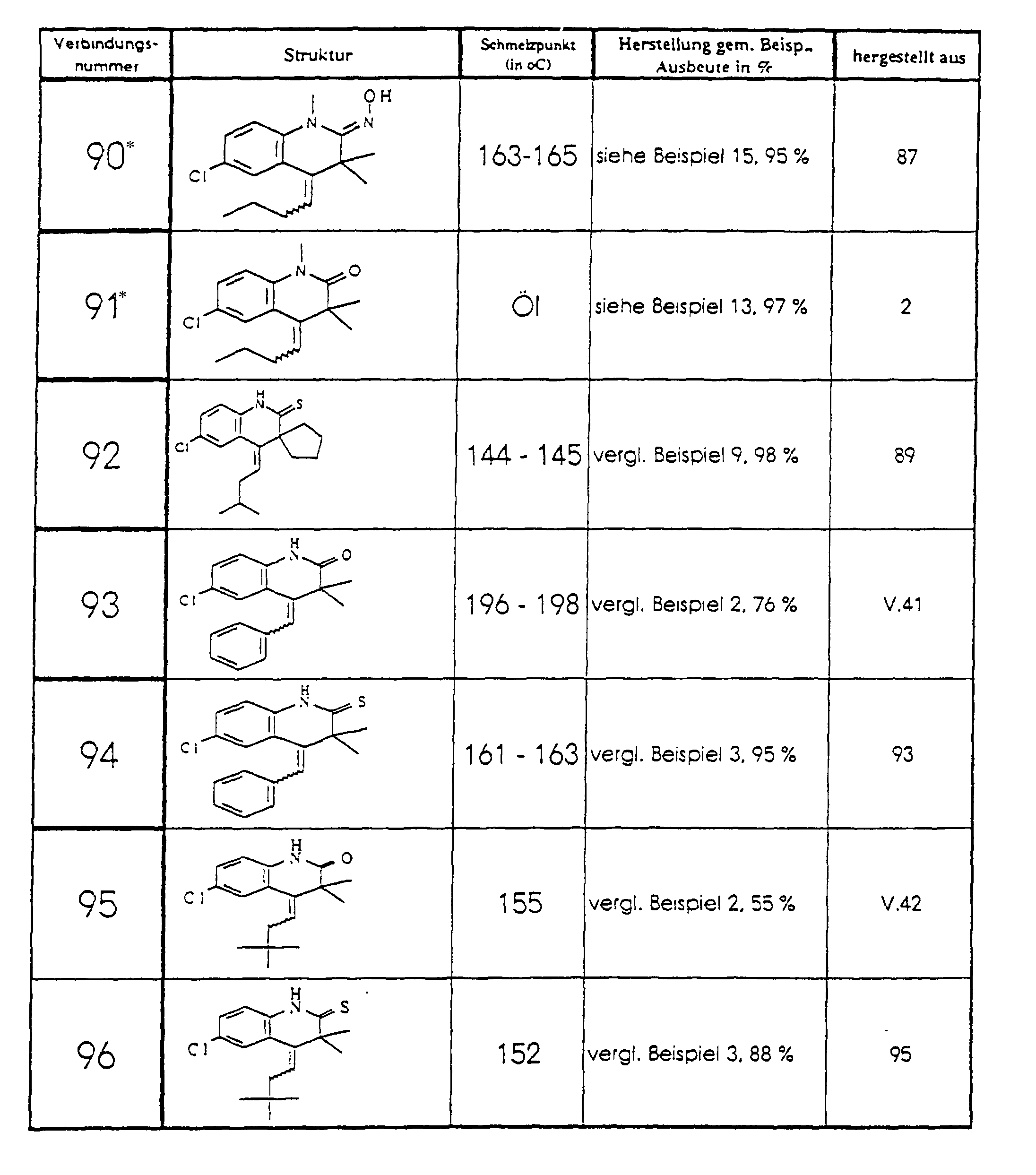

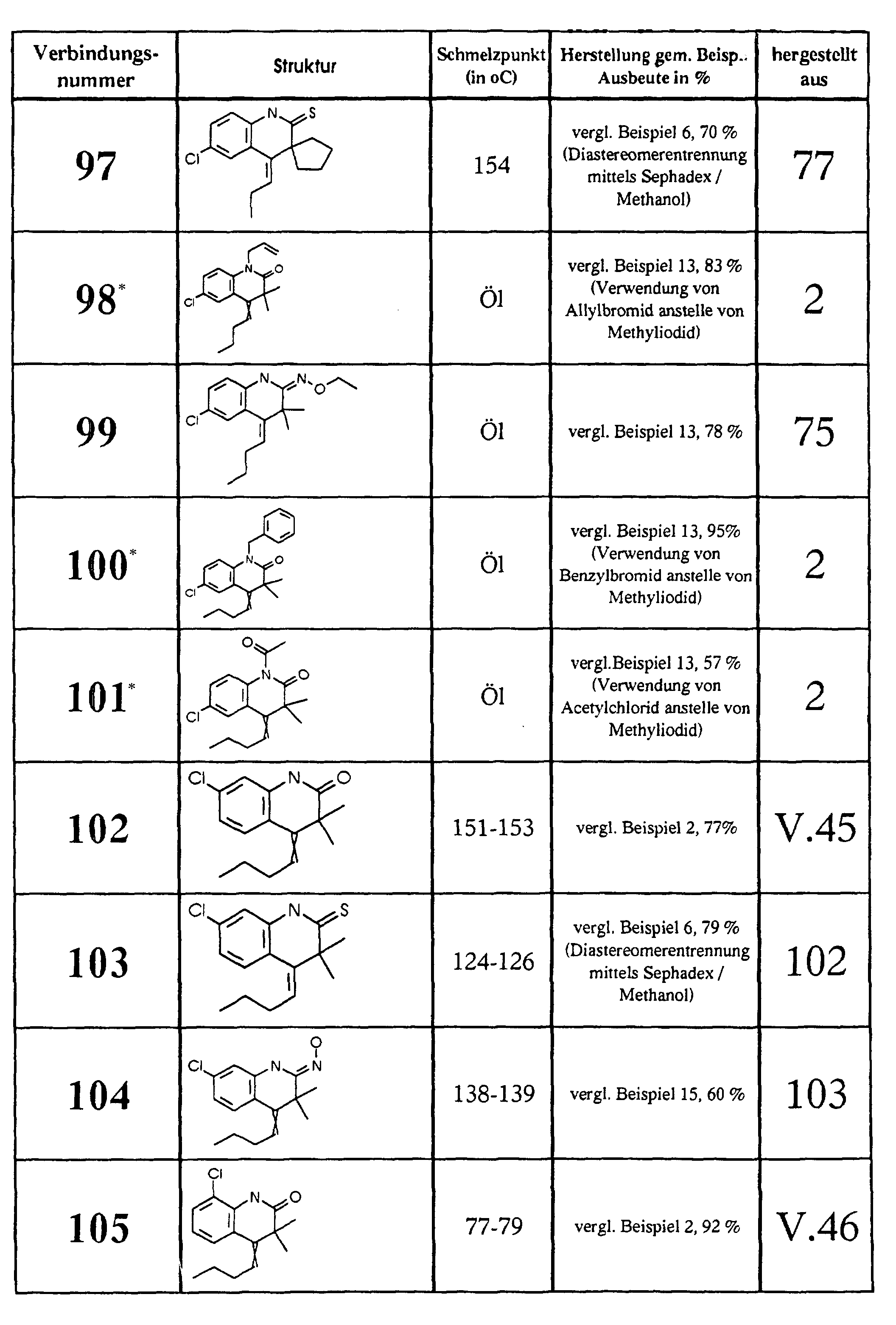

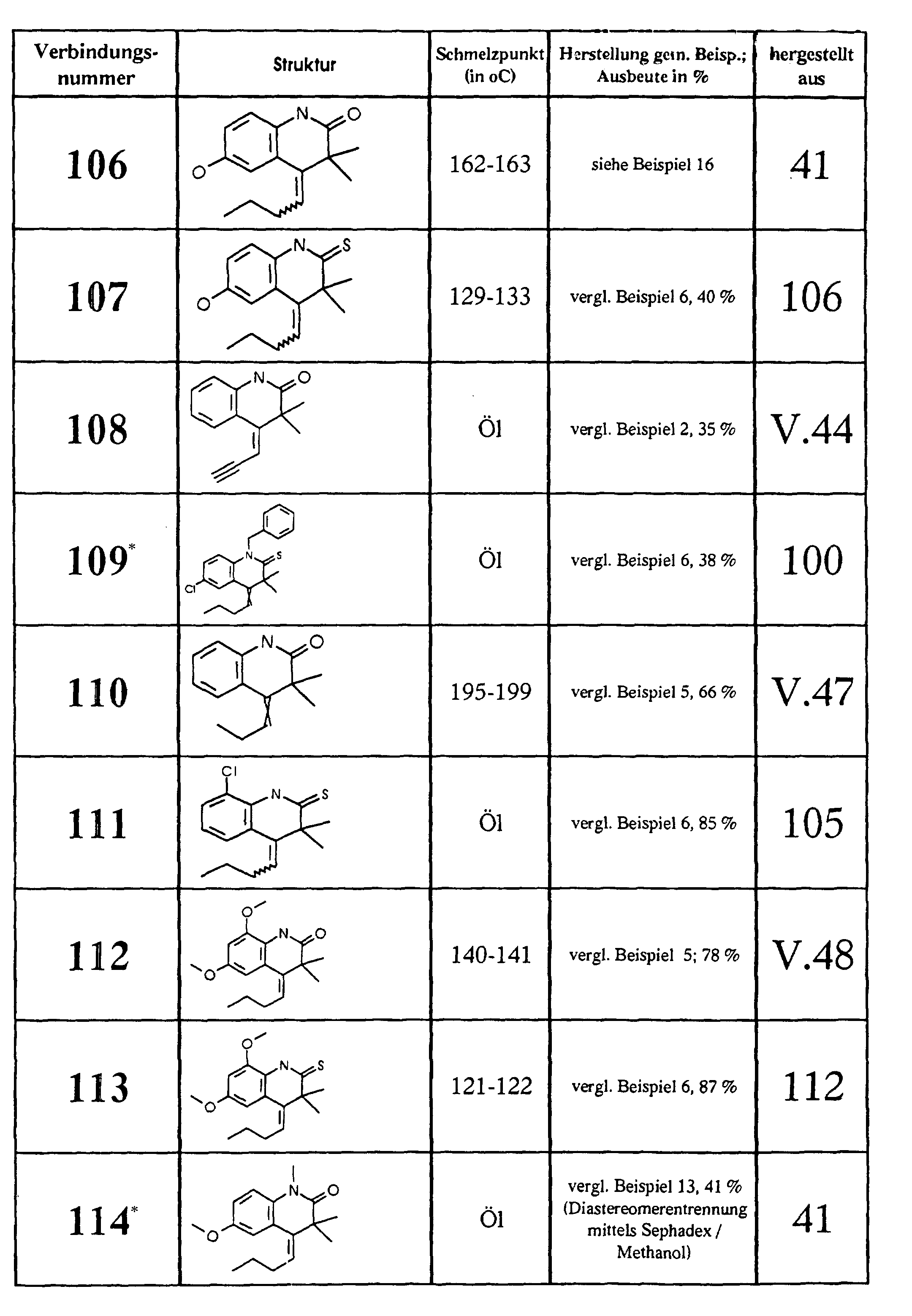

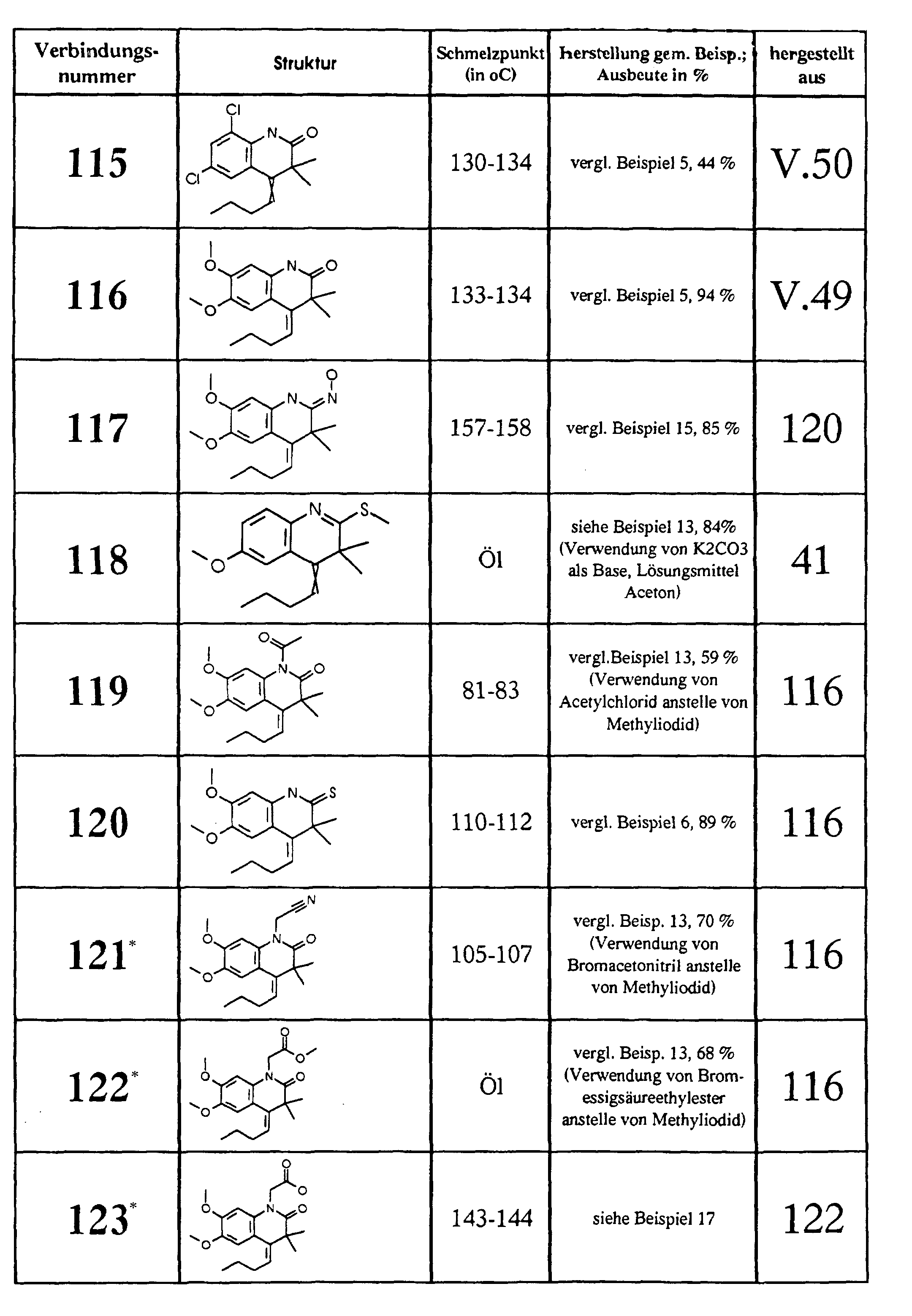

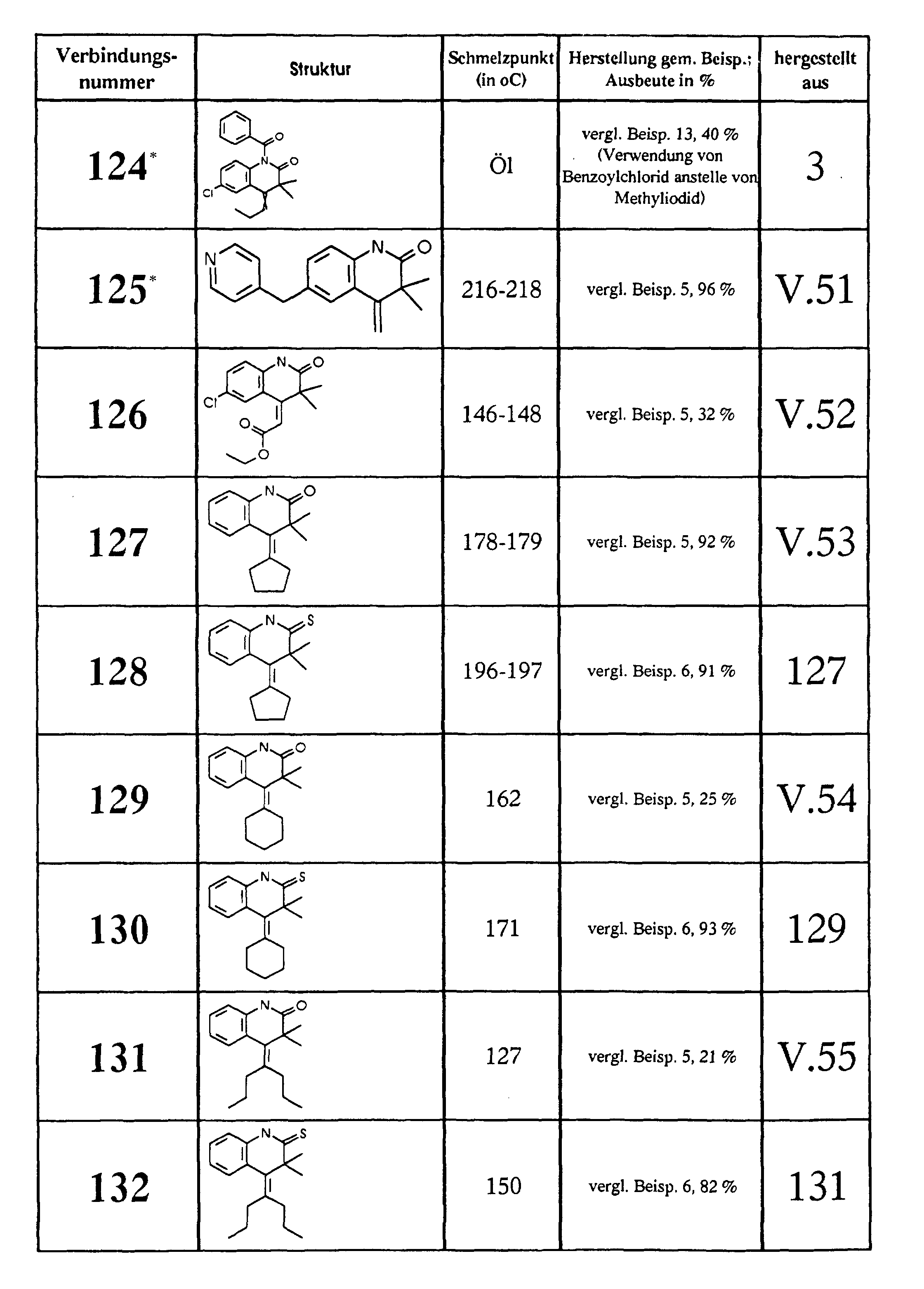

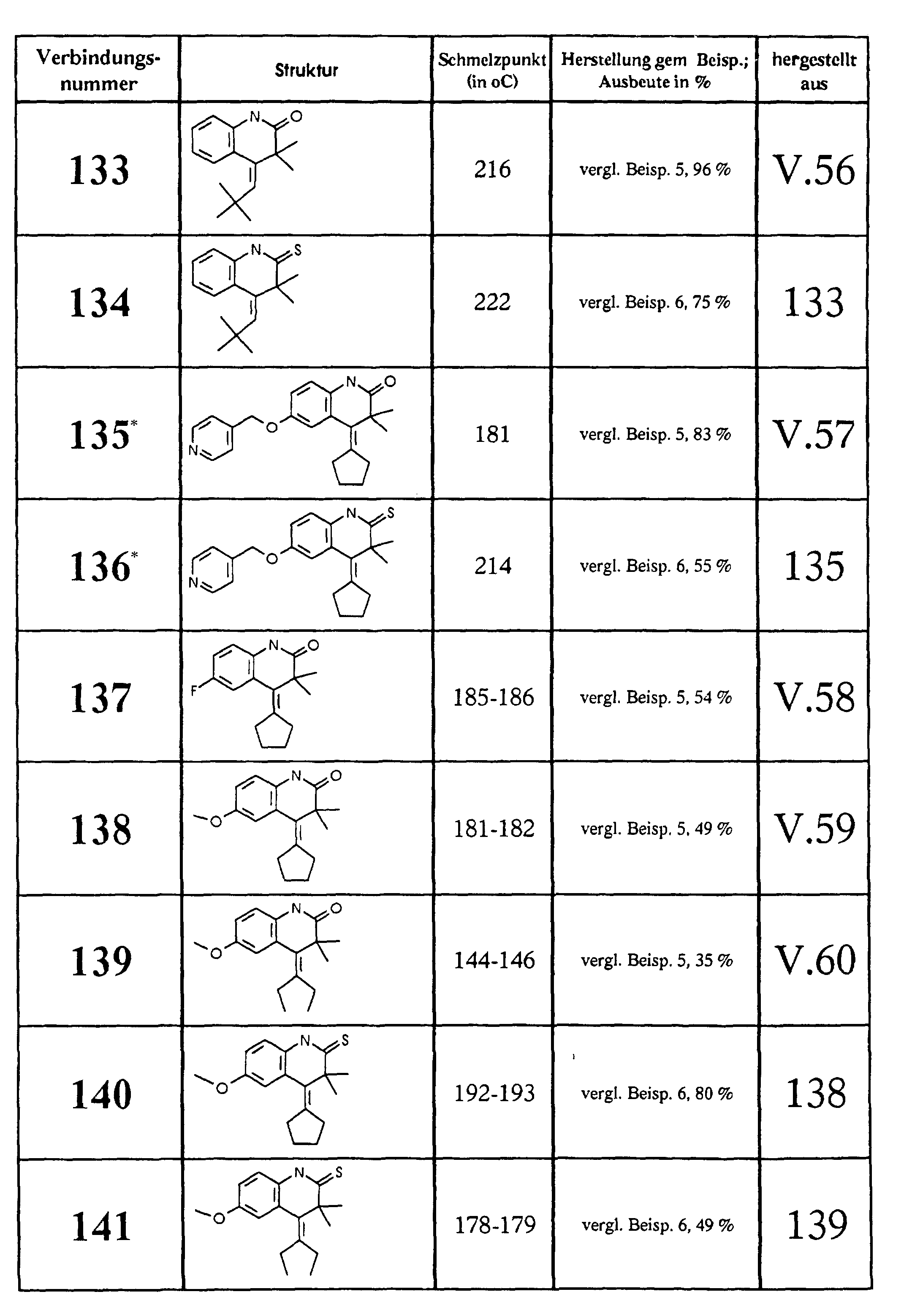

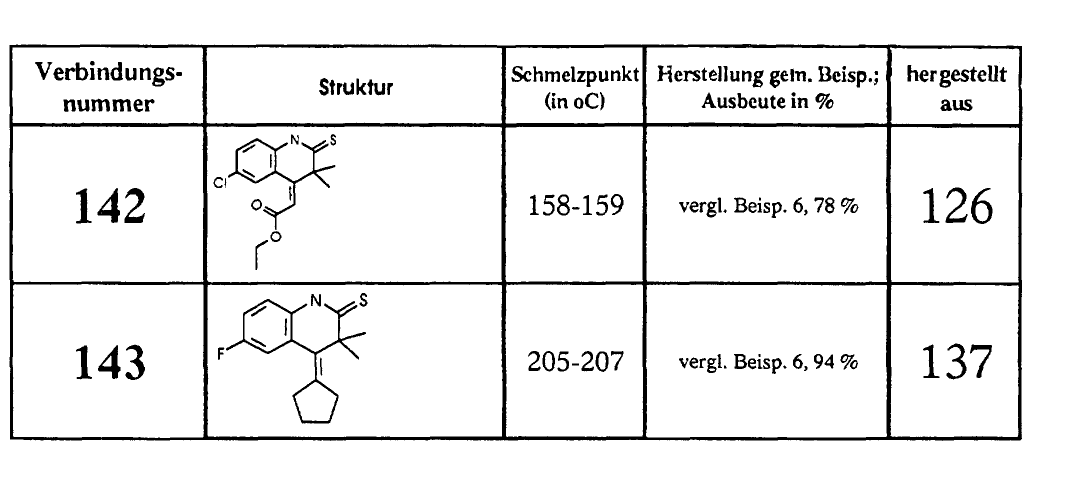

Die Herstellung der in Tabelle 3 aufgeführten Derivate werden i.a. aus den betreffenden Verbindungen der Formel II oder lla mit Z gleich Hydroxy. Tabelle 4 gibt eine Übersicht aller bislang synthetisierten Vorprodukte wieder.
Die als Edukte bei Herstellung der Hydroxyverbindungen verwendeten entsprechenden substituierten Chinolindione können nach den Methoden der Beispiele I - III hergestellt werden.

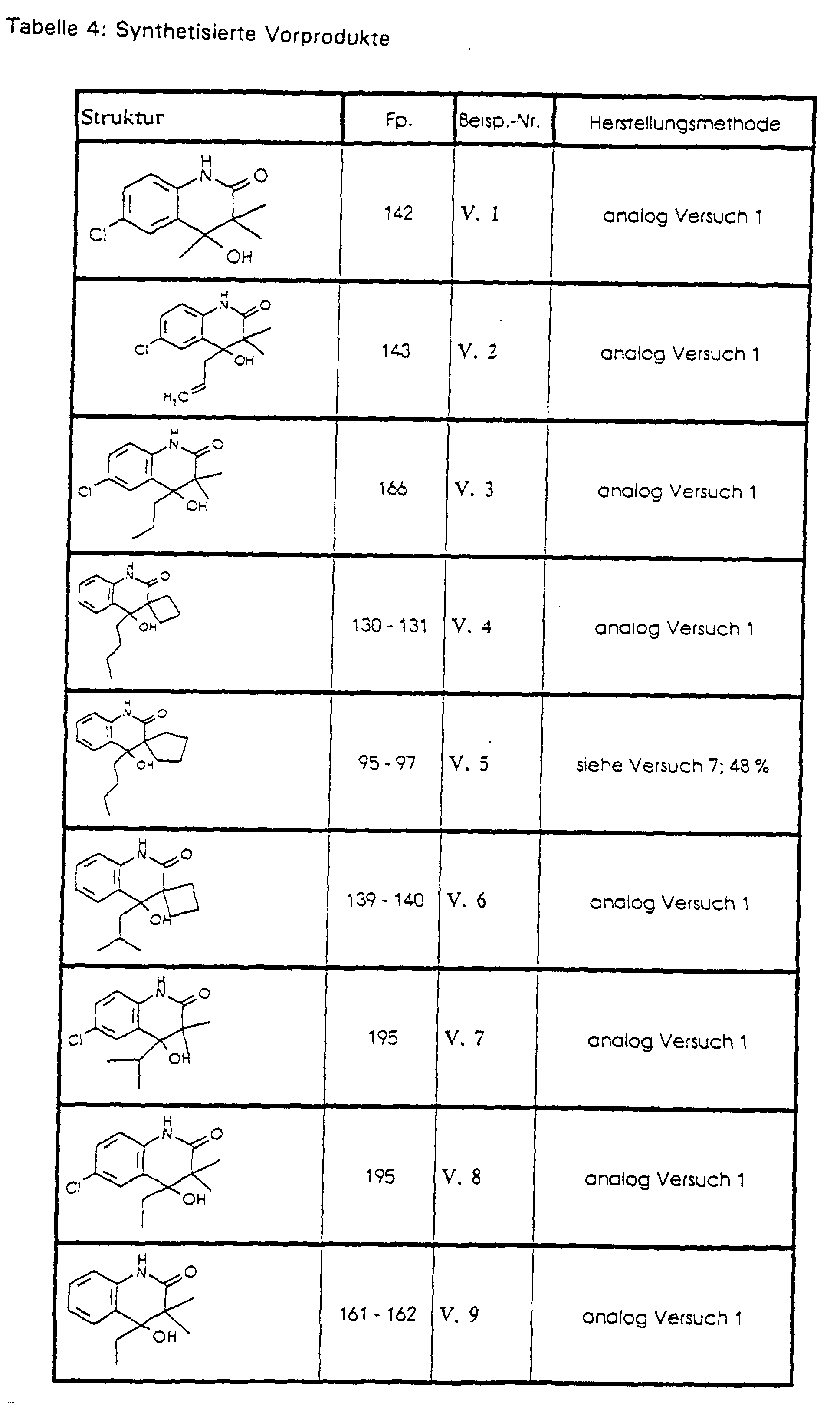

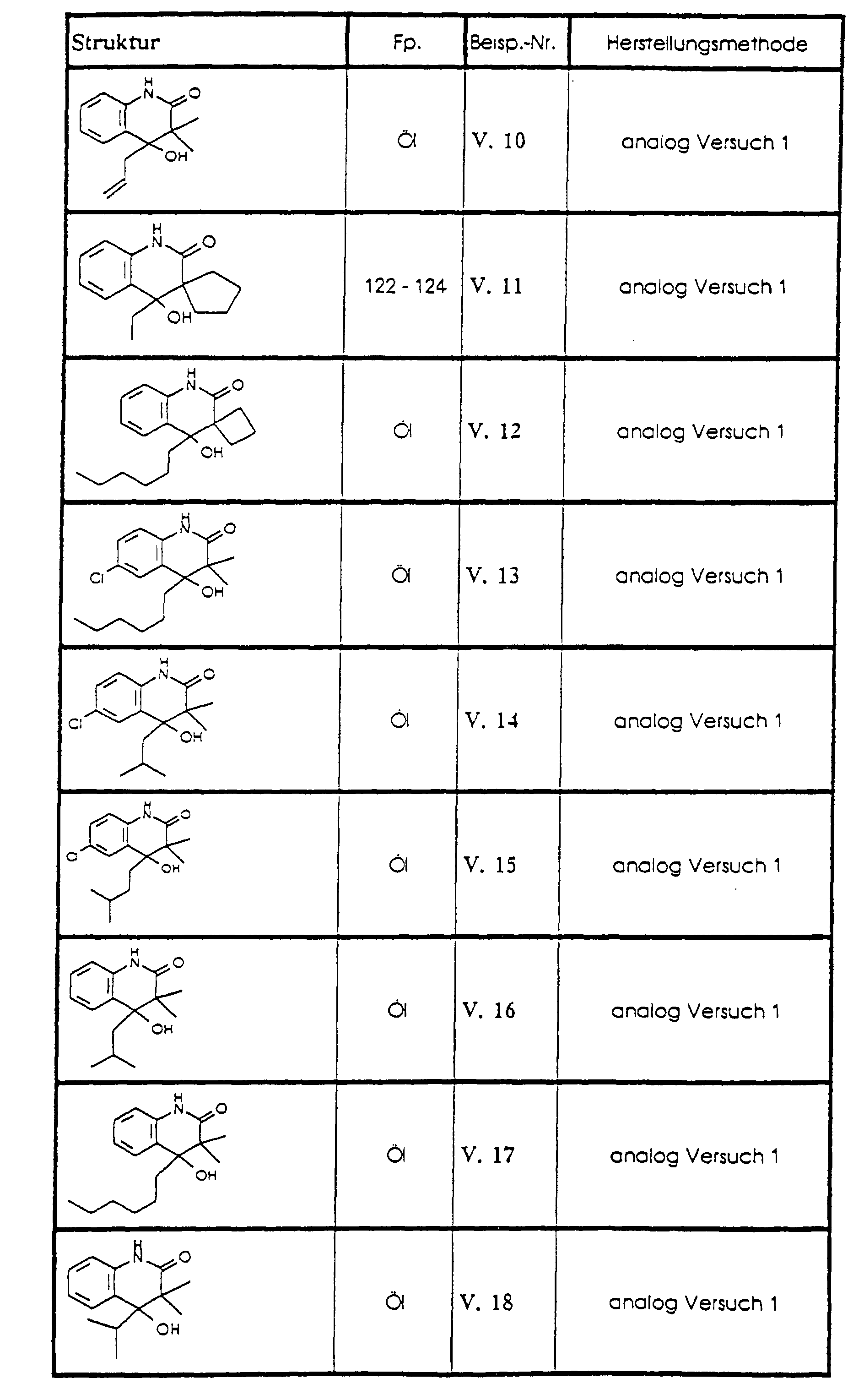

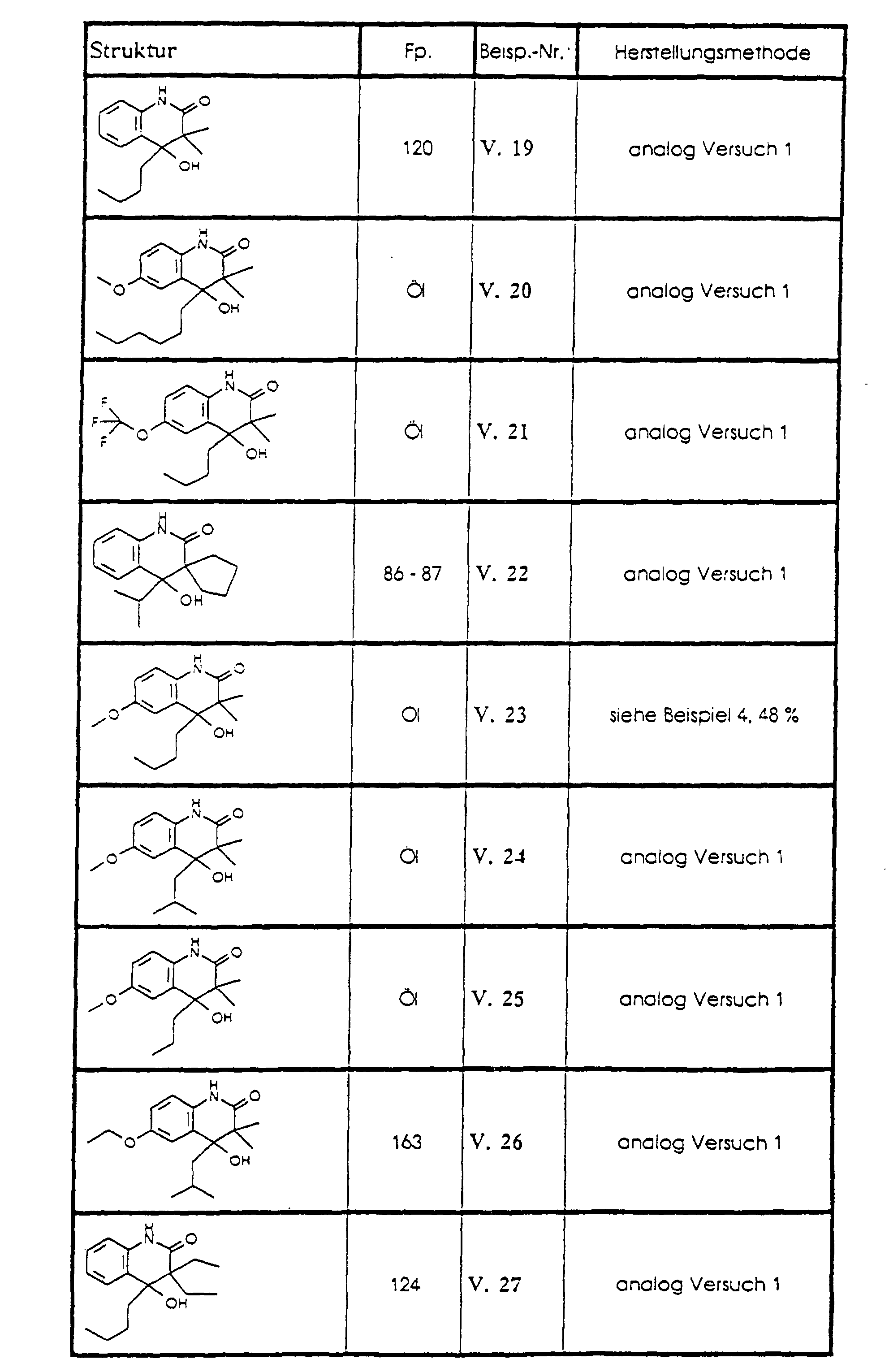

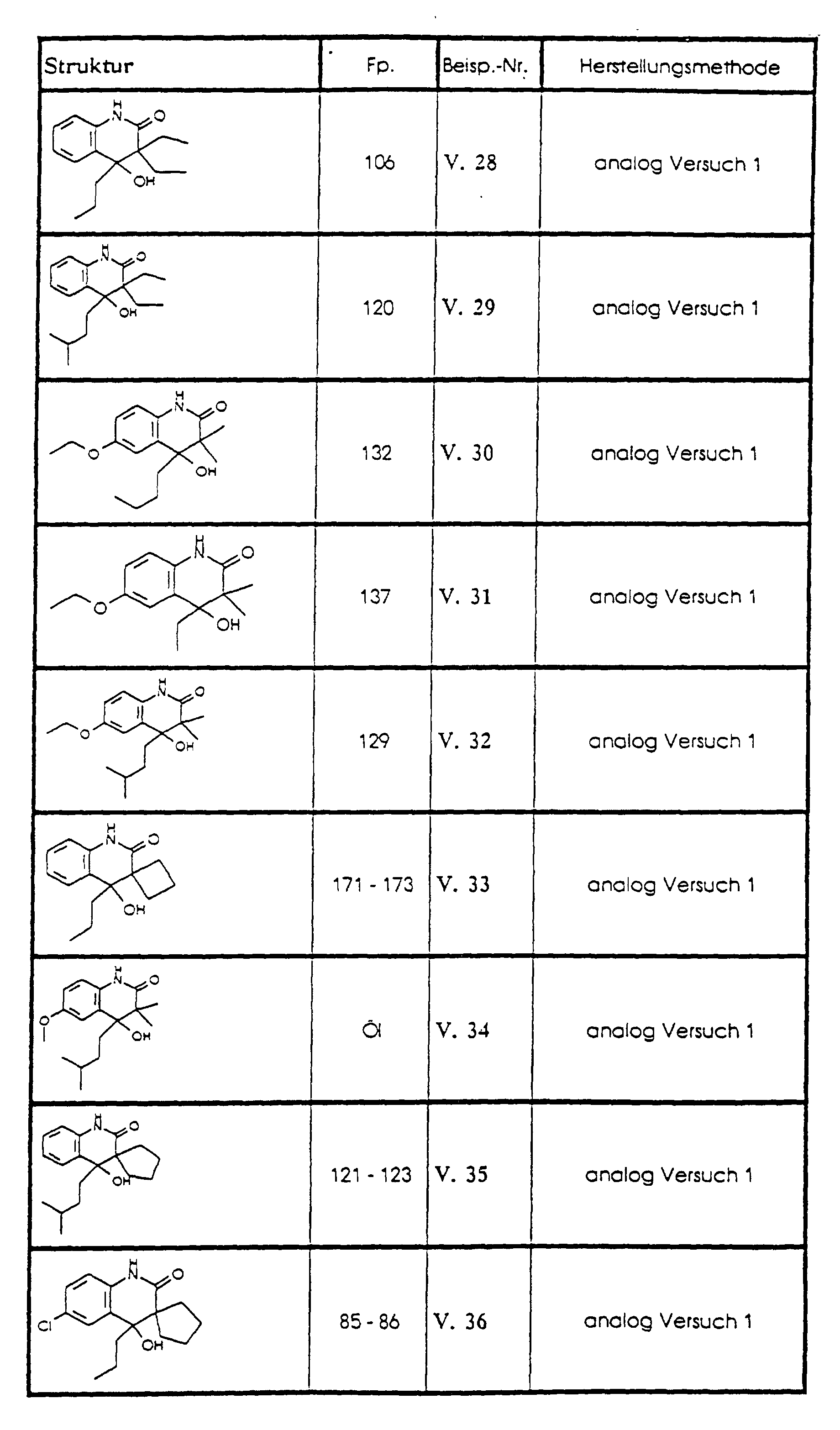

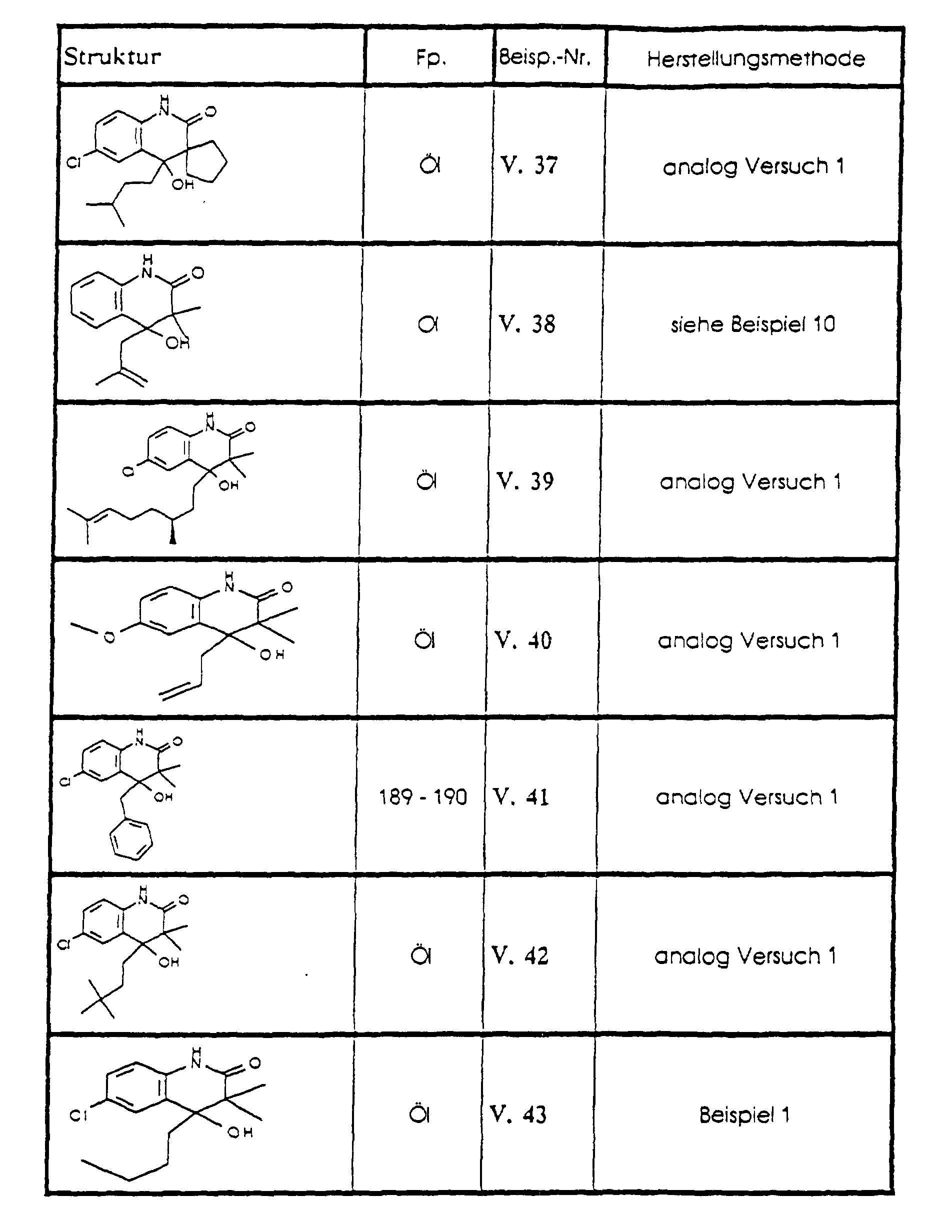

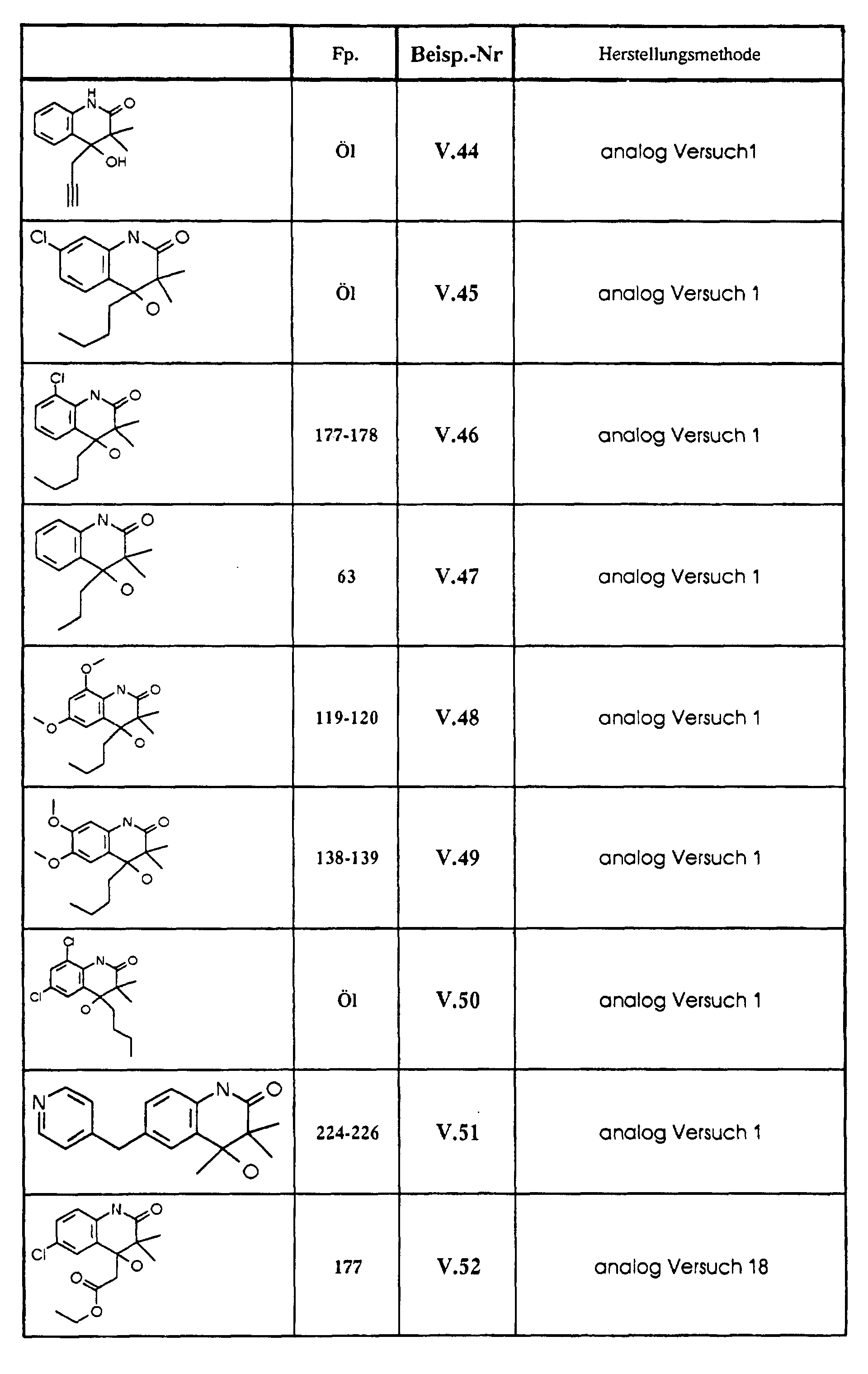

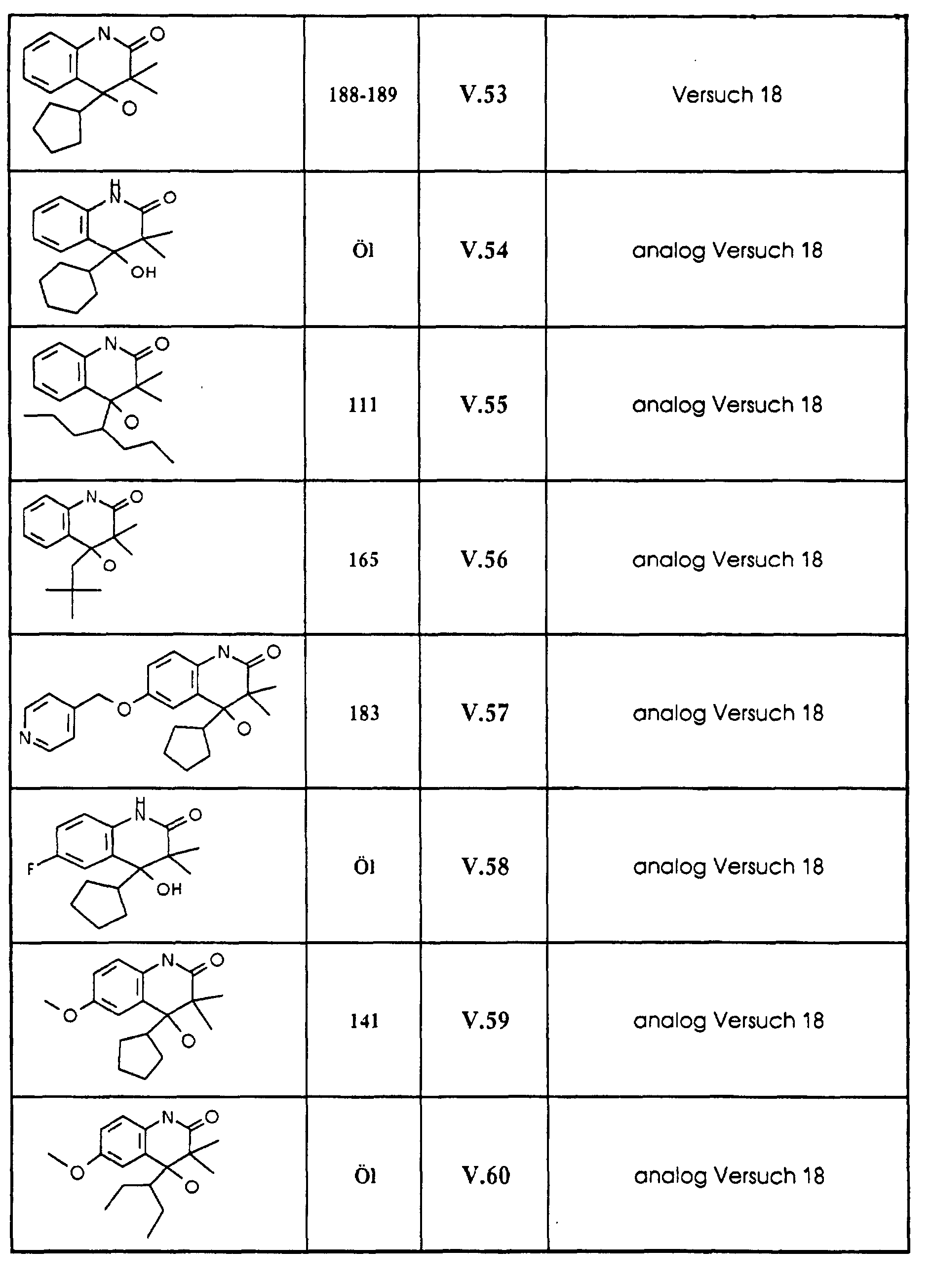

## Patentansprüche

1. Verbindungen der Formel I, 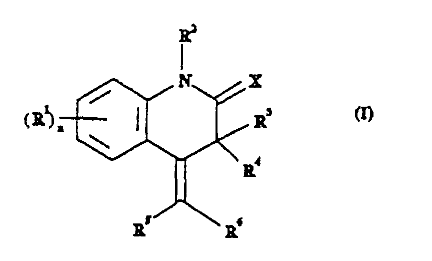 sowie deren tautomere Formen, der allgemeinen Formel la, 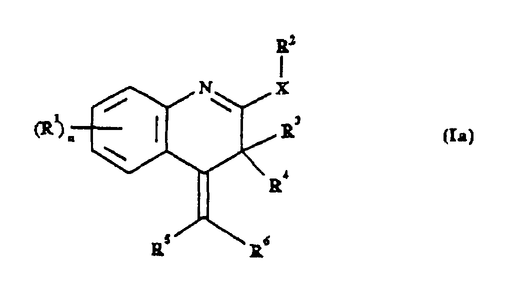 worin bedeuten:
n
null,
eins,
oder zwei,
die einzelnen Substituenten R¹ unabhängig voneinander Fluor,
Chlor, Trifluormethoxy, Hydroxy, C₁-C₄-Alkoxy, C₁-C₄-Alkylthio,
X Sauerstoff, Schwefel oder substituierten Stickstoff N-R², N-O-R²,
wobei R² Wasserstoff bedeutet;
R⁵ und R⁶ gleich oder verschieden, unabhängig voneinander Wasserstoff,
C₁-C₆-Alkyl, gegebenenfalls substituiert mit Fluor, Chlor, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio;
C₂-C₆-Alkenyl, gegebenenfalls substituiert mit Fluor, Chlor, Amino, Mercapto, Hydroxy, C₁-C₄-Acyloxy, Benzoyloxy, Benzyloxy, Phenoxy, C₁-C₄-Alkoxy, C₁-C₄-Alkylamino, Di(C₁-C₄-alkyl)amino, C₁-C₄-Alkylthio;
R⁵ und R⁶ können auch gemeinsam einen Carbozyklus der Ringgröße C₅-C₆ bedeuten, welcher über die Doppelbindung mit dem Chinolinsystem verknüpft ist
und
R³ und R⁴ gleich oder verschieden, unabhängig voneinander C₁-C₂-Alkyl;
R³ und R⁴ können auch in Strukturen der Formeln gemäß I und la gemeinsam einen Carbozyklus der Ringgröße C₄-C₆ bedeuten, welcher spiroartig mit dem Chinolinsystem verknüpft ist.
deren optische Isomere, Diastereomere in reiner Form oder in Form ihrer Mischungen und deren Additionssalze.

2. Verbindungen der Formeln I oder la gemäß Anspruch 1 zur Anwendung als Arzneimittel.

3. Verwendung von Verbindungen der Formeln I oder la gemäß Anspruch 1 zur Herstellung von Arzneimitteln zur Bekämpfung von Viruserkrankungen.

4. Arzneimittel enthaltend eine oder mehrere Verbindungen gemäß Anspruch 1.

5. Verfahren zur Herstellung von Arzneimitteln, **dadurch gekennzeichnet, daß** eine oder mehrere Verbindungen gemäß Anpruch 1, ggf. mit üblichen Hilfs- und/oder Trägerstoffen in eine geeignete Darreichungsform gebracht werden.

6. Verfahren zur Herstellung von Verbindungen der Formeln I oder la gemäß Anspruch 1, **dadurch gekennzeichnet, daß**
A) zur Herstellung von Verbindungen der Formeln I mit X gleich Sauerstoff und la mit X wie in Anspruch 1 definiert - mit der Ausnahme von N-R² gleich N-H - und den Resten R¹, R², R³, R⁴, R⁵ und R⁶ wie in Anspruch 1 definiert,
man eine Verbindung der Formel II und IIa , 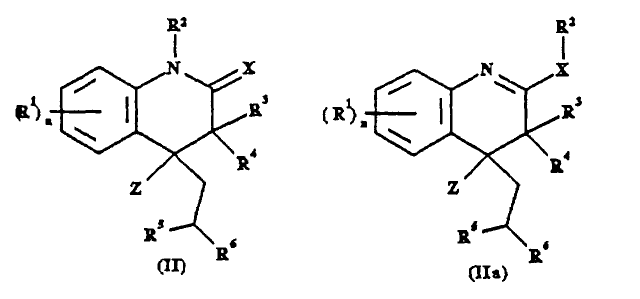 , wobei Z
eine Abgangsgruppe oder eine Hydroxylgruppe darstellt, in einem inerten Lösungsmittel erhitzt, gegebenfalls unter Zusatz eines sauren oder basischen Katalysators,
B) zur Herstellung von Verbindungen der Formeln II mit X gleich Sauerstoff und Z gleich Hydroxyl und lla Z gleich Hydroxyl, mit X wie in Anspruch 1 definiert - mit der Ausnahme von N-R² gleich N-H - und den Resten R¹, R², R³, R⁴, R⁵ und R⁶ wie in Anspruch 1 definiert
eine Verbindung der Formel III und IIIa, 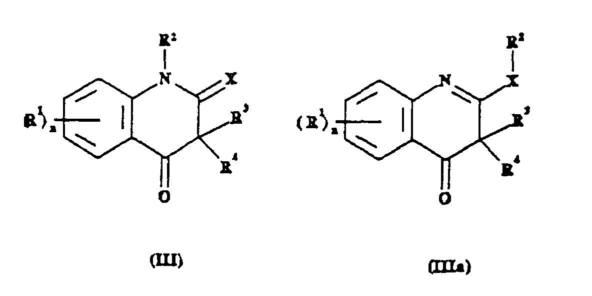 umsetzt mit einer Verbindung der Formel IV 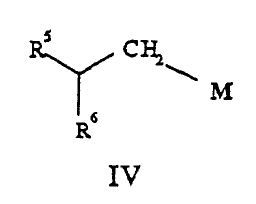 wobei M ein Metallatomäquivalent wie Li, -MgCl, -MgBr ist,
oder
C) zur Herstellung von Verbindungen der Formeln I mit X gleich Schwefel und R¹, R², R³, R⁴, R⁵ und R⁶ wie in Anspruch 1 definiert, durch Reaktion einer Verbindung der Formel I , wobei X gleich Sauerstoff ist und für R¹, R², R³, R⁴, R⁵ und R⁶ die in Anspuch 1 genannten Definitionen gelten, mit einem Schwefelungsreagenz
oder daß
D) Verbindungen der Formel I mit X gleich Sauerstoff, R² gleich Wasserstoff und R¹, R³, R⁴, R⁵ und R⁶ wie in Anspruch 1 definiert, hergestellt werden durch Reaktion mit einem Alkylierungsreagenz der Formel V
R²-K, V
wobei R² die in Anspruch 1 genannten Bedeutungen mit Ausnahme von R² gleich Wasserstoff aufweist und die Abgangsgruppe K beispielsweise ein Halogenatom wie Chlor oder Brom oder eine Sulfonsäureestergruppen wie Mesylat oder Triflat darstellt,
oder daß
E) man eine Verbindung der Formel I mit R¹ - R⁶ wie in Ansprüch 1 definiert und X ein Sauerstoff- oder Schwefelatom ist umsetzt mit einer Verbindung der Formel
R²-NH₂ oder R²-O-NH₂
zu Derivaten der Formel I mit R¹ - R⁶ wie in Anspruch 1 definiert und X gleich N-R² oder N-O-R²,
F) man eine Verbindung der Formel I mit R¹ - R⁶ wie in Anspruch 1 definiert und dabei einer dieser Reste eine Alkoxycarbonylgruppe besitzt, umsetzt mit einer Verbindung der Formel
Met-OH,
mit Met gleich einen Alkali- oder erdalkalimetallatom ist, zu derivaten der Formel I, die eine freie Carbonsäurefunktion aufweisen,
oder daß
G) man eine Verbindung der Formel I mit R¹ = Methoxy und R² - R⁶ wie in den Anspruch 1 definiert und X Sauerstoff umsetzt, mit Trimethylsilyliodid zu einer Verbindung der Formel I mit R¹ = Hydroxy und den Resten R² - R⁶ bzw. X wie oben definiert.

## Claims

1. A compound of the formula I, 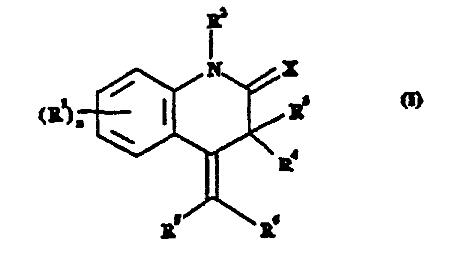 and also one of its tautomeric forms of the formula Ia, 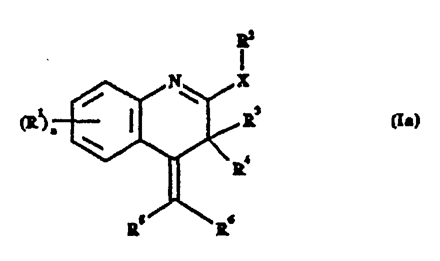 in which:
n is zero, one or two,
the individual substituents R¹ are, independently of each other, fluorine, chlorine, trifluoromethoxy, hydroxyl, C₁-C₄-alkoxy or C₁-C₄-alkylthio,
X is oxygen, sulfur or substituted nitrogen N-R² or N-O-R²,
where R² is hydrogen;
R⁵ and R⁶ are identical or different and are, independently of each other, hydrogen,
C₁-C₆-alkyl which is optionally substituted by fluorine, chlorine, amino, mercapto, hydroxyl, C₁-C₄-acyloxy, benzoyloxy, benzyloxy, phenoxy, C₁-C₄-alkoxy, C₁-C₄-alkylamino, di(C₁-C₄-alkyl)amino or C₁-C₄-alkylthio;
C₂-C₆-alkenyl which is optionally substituted by fluorine, chlorine, amino, mercapto, hydroxyl, C₁-C₄-acyloxy, benzoyloxy, benzyloxy, phenoxy, C₁-C₄-alkoxy, C₁-C₄-alkylamino, di(C₁-C₄-alkyl)amino or C₁-C₄-alkylthio;
R⁵ and R⁶ can also together be a carbocycle which is of a ring size of C₅-C₆ and which is linked to the quinoline system via the double bond,
and
R³ and R⁴ are identical or different and are, independently of each other, C₁-C₂-alkyl;
R³ and R⁴ can also, in structures of the formulae I and Ia, together be a carbocycle which is of a ring size of C₄-C₆ and which is linked to the quinoline system in a spiro manner,
their optical isomers and diastereomers in pure form or in the form of their mixtures, and their addition salts.

2. A compound of the formula I or Ia as claimed in claim 1 for use as a pharmaceutical.

3. The use of compounds of the formulae I or Ia as claimed in claim 1 for preparing pharmaceuticals for treating viral diseases.

4. A pharmaceutical containing one or more compounds as claimed in claim 1.

5. A process for preparing pharmaceuticals, wherein one or more compounds as claimed in claim 1, where appropriate together with customary auxiliary and/or carrier substances, is/are brought into a suitable form for administration.

6. A process for preparing compounds of the formulae I or Ia as claimed in claim 1, wherein
A) in order to prepare compounds of the formulae I in which X is oxygen and Ia in which X is defined as in claim 1 - with the exception of N-R² being N-H - and the radicals R¹, R², R³, R⁴, R⁵ and R⁶ are defined as in claim 1,
a compound of the formula II and IIa, 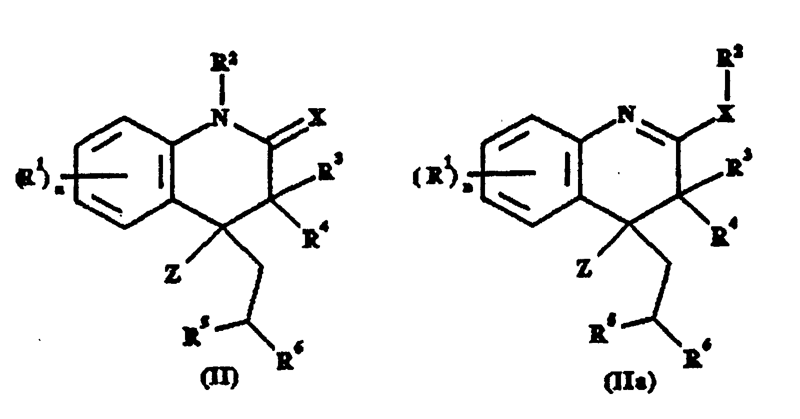 in which Z is a leaving group or a hydroxyl group, is heated in an inert solvent, where appropriate with an acidic or basic catalyst being added,
B) in order to prepare compounds of the formulae II in which X is oxygen and Z is hydroxyl and IIa in which Z is hydroxyl, X is defined as in claim 1 - with the exception of N-R² being N-H - and the radicals R¹, R², R³, R⁴, R⁵ and R⁶ are defined as in claim 1,
a compound of the formula III or IIIa, 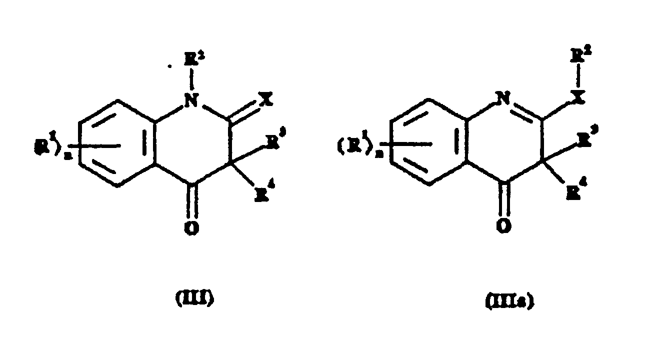 is reacted with a compound of the formula IV 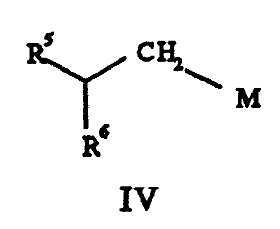 where M is a metal atom equivalent such as Li, -MgCl or -MgBr,
or
C) in order to prepare compounds of the formulae I in which X is sulfur and R¹, R², R³, R⁴, R⁵ and R⁶ are defined as in claim 1, by means of reacting a compound of the formula I, where X is oxygen and the definitions mentioned in claim 1 apply to R¹, R², R³, R⁴, R⁵ and R⁶, with a sulfurization reagent, or wherein
D) compounds of the formula I in which X is oxygen, R² is hydrogen and R¹, R³, R⁴, R⁵ and R⁶ are defined as in claim 1, are prepared by reacting with an alkylating reagent of the formula V
R²-K V
where R² has the meanings given in claim 1, with the exception of R² being hydrogen, and the leaving group K is, for example, a halogen atom such as chlorine or bromine or is a sulfonic ester group such as mesylate or triflate,
or wherein
E) a compound of the formula I in which R¹-R⁶ are defined as in claim 1 and X is an oxygen atom or a sulfur atom is reacted with a compound of the formula
R²-NH₂ or R²-O-NH₂
to form derivatives of the formula I in which R¹-R⁶ are defined as in claim 1 and X is N-R² or N-O-R²,
or wherein
F) a compound of the formula I in which R¹-R⁶ are defined as in claim 1, with one of these radicals possessing an alkoxycarbonyl group, is reacted with a compound of the formula
Met-OH,
in which Met is an alkali metal atom or alkaline earth metal atom, to form derivatives of the formula I which possess a free carboxylic acid function,
or wherein
G) a compound of the formula I in which R¹ = methoxy and R²-R⁶ are defined as in claim 1 and X is oxygen is reacted with trimethylsilyl iodide to form a compound of the formula I in which R¹ = hydroxyl and the radicals R²-R⁶ and X are defined as above.

## Revendications

1. Composés de formule I, 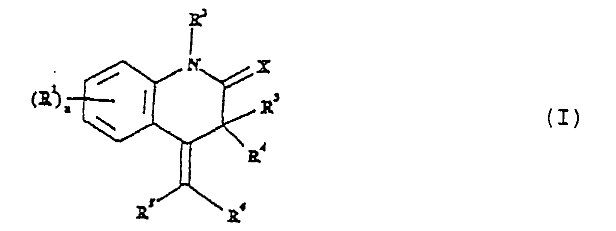 ainsi que leurs formes tautomères de formule générale Ia 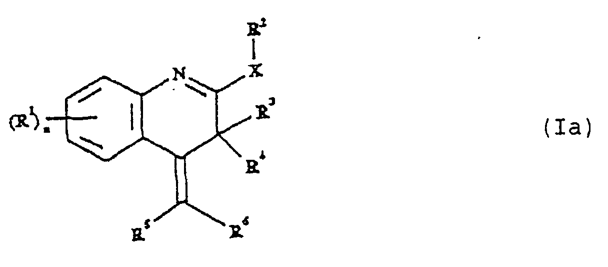 où
n vaut zéro ou un ou deux,
les différents substituants
R¹ représentent, indépendamment l'un de l'autre, des atomes de fluor, de chlore, des groupes trifluorométhoxy, hydroxy, alkoxy en C₁-C₄, (alkyl en C₁-C₄)thio,
X représente un atome d'oxygène, un atome de soufre ou un atome d'azote substitué N-R², N-O-R², où R² représente un atome d'hydrogène ;
R⁵ et R⁶ représentent, identiques ou différents, indépendamment l'un de l'autre, des atomes d'hydrogène, des groupes alkyle en C₁-C₆, éventuellement substitué par des substituants fluoro, chloro, amino, mercapto, hydroxy, acyloxy en C₁-C₄, benzoyloxy, benzyloxy, phénoxy, alkoxy en C₁-C₄, (alkyl en C₁-C₄)amino, di(alkyl en C₁-C₄)amino, (alkyl en C₁-C₄)thio ;
alcényle en C₂-C₆, éventuellement substitué par des substituants fluoro, chloro, amino, mercapto, hydroxy, acyloxy en C₁-C₄, benzoyloxy, benzyloxy, phénoxy, alkoxy en C₁-C₄, (alkyl en C₁-C₄)amino, di(alkyl en C₁-C₄)amino, (alkyl en C₁-C₄)thio ;
R⁵ et R⁶ peuvent également représenter conjointement un cycle carboné d'une taille de cycle en C₅-C₆, qui est relié par la double liaison au système quinoléine,
et
R³ et R⁴ représentent, identiques ou différents, indépendamment l'un de l'autre, un groupe alkyle en C₁-C₂ ;
R³ et R⁴ peuvent représenter conjointement, dans les structures selon les formules I et Ia, un cycle carboné d'une taille en C₄-C₆ qui peut être relié au système quinoléine par une liaison de type spirannique,
leurs isomères optiques, leurs diastéréomères sous forme pure ou sous forme de mélanges et leurs sels d'addition.

2. Composés de formules I ou Ia selon la revendication 1 pour application comme médicaments.

3. Composés de formules I ou Ia selon la revendication 1 pour la préparation de médicaments pour la lutte contre des maladies virales.

4. Médicament contenant un ou plusieurs composés selon la revendication 1.

5. Procédé pour la préparation de médicaments, **caractérisé en ce qu'**on met en forme d'administration appropriée un ou plusieurs composés selon la revendication 1, éventuellement avec des adjuvants et véhicules usuels.

6. Procédé pour la préparation de composés de formules I ou Ia selon la revendication 1, **caractérisé en ce que**
A) pour préparer des composés de formules I avec et Ia avec X défini comme à la revendication 1 - à l'exception de N-R² égal à N-H - et les restes R¹, R², R³, R⁴, R⁵ et R⁶ sont définis comme à la revendication 1,
on chauffe un composé de formule II et IIa, 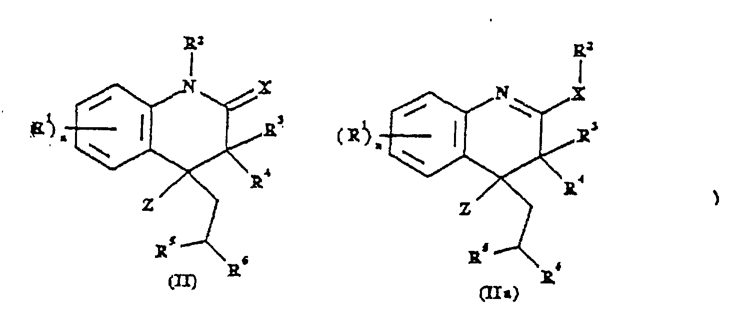 où Z représente un groupe partant ou un groupe hydroxyle, dans un solvant inerte, éventuellement sous ajout d'un catalyseur acide ou basique,
B) pour préparer des composés de formule II, où X représente un atome d'oxygène et Z représente un groupe hydroxyle, et de formule IIa, où Z représente un groupe hydroxyle, où X est défini comme à la revendication 1) - à l'exception de N-R² égal à N-H - et les restes R¹, R², R³, R⁴, R⁵ et R⁶ sont définis comme à la revendication 1, on fait réagir un composé de formules III et IIIa 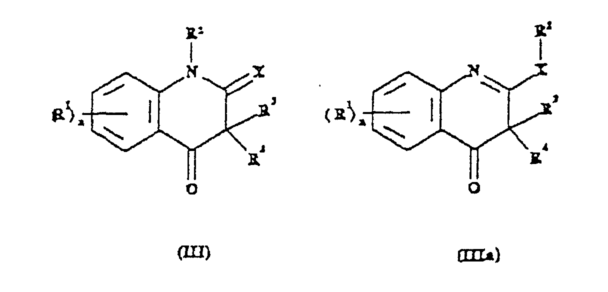 sur un composé de formule IV 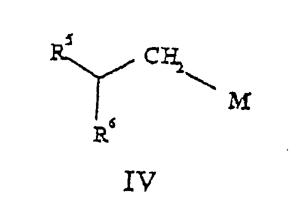 M représentant un équivalent d'atome métallique comme Li, -MgCl, -MgBr,
ou
C) pour préparer des composés de formules I, où X représente un atome de soufre et R¹, R², R³, R⁴, R⁵ et R⁶ sont définis comme à la revendication 1), par réaction d'un composé de formule I, où X représente un atome d'oxygène et les définitions citées à la revendication 1) s'appliquent à R¹, R², R³, R⁴, R⁵ et R⁶, sur un réactif soufrés, ou
**en ce que**
D) on prépare des composés de formule I, où X représente un atome d'oxygène, R² représente un atome d'hydrogène et R¹, R², R³, R⁴, R⁵ et R⁶ sont définis comme à la revendication 1), par réaction sur un réactif d'alkylation de formule V
R²-K (V),
où R² présente les significations données à la revendication 1), à l'exception de R² égal à un atome d'hydrogène et le groupe partant K représente, par exemple un atome d'halogène comme le chlore ou le brome ou un groupe ester d'acide sulfonique comme mésylate ou triflate,
ou **en ce que**
E) on fait réagir un composé de formule I, où R¹ à R⁶ définis comme à la revendication 1) et X représente un atome d'oxygène ou de soufre, sur un composé de formule
R²-NH₂ ou R²-O-NH₂
pour obtenir des dérivés de formule I, où R¹ à R⁶ sont définis comme à la revendication 1) et X représente des groupes N-R² ou N-O-R²,
F) on fait réagir un composé de formule I, où R¹ à R⁶ définis comme à la revendication 1), et pour ce faire, un de ces restes porte un groupe alkoxycarbonyle, sur un composé de formule
Met-OH,
où Met représente un atome de métal alcalin ou alcalino-terreux, pour obtenir des dérivés de formule I, qui présentent une fonction acide carboxylique libre,
ou **en ce que**
G) on fait réagir un composé de formule I où R¹ = méthoxy et R² à R⁶ sont définis comme à la revendication 1), et X représente un atome d'oxygène, sur l'iodure de triméthylsilyle, pour donner un composé de formule I, où R¹ = hydroxy et les restes R² à R⁶ ou définis comme ci-dessus.
